# EUROPEAN PATENT APPLICATION

(11) **EP 4 807 749 A1**
(43) Date of publication of application: **16.09.2026**
(21) Application number: 23958337.0
(22) Date of filing: 10.11.2023
(51) Int. Cl.: G16B 15/00

(54) **INFORMATION PROCESSING PROGRAM, INFORMATION PROCESSING METHOD, AND INFORMATION PROCESSING DEVICE**

(71) Applicant: FUJITSU LIMITED, Kawasaki-shi, Kanagawa 211-8588 (JP)
(72) Inventor: ISHIZAKI, Ryo, Kawasaki-shi, Kanagawa 211-8588 (JP); KITAJIMA, Masato, Kawasaki-shi, Kanagawa 211-8588 (JP); KURIBAYASHI, Sotaro, Kawasaki-shi, Kanagawa 211-8588 (JP); HIGUCHI, Hiroyuki, Kawasaki-shi, Kanagawa 211-8588 (JP)
(74) Representative: Hoffmann Eitle
(86) International application number: PCT/JP2023/040612
(87) International publication number: WO 2025/099938

(57) **Abstract**

To efficiently determine whether a predetermined relationship exists between amino acids.

An information processing apparatus calculates, for each of a plurality of amino acid pairs each being a combination of two amino acids among a plurality of amino acids, a distance between the combined amino acids. The information processing apparatus then determines, in response to the distance between the combined amino acids being equal to or greater than a threshold, that the predetermined relationship does not exist between the combined amino acids. The information processing apparatus calculates, in response to the distance between the combined amino acids being less than the threshold, an inter-atomic distance between an atom constituting one amino acid of the combined amino acids and an atom constituting another amino acid of the combined amino acids, and determines, based on the inter-atomic distance, whether the predetermined relationship exists between the combined amino acids.

## Description

### Technical Field

Embodiments of the present disclosure relate to an information processing program, an information processing method, and an information processing apparatus.

### Background Art

Based on three-dimensional structural information of amino acids constituting a protein, many findings relating to the protein may be obtained. For example, by performing a prediction task such as discrimination of types of virus strains (antigen clusters), analysis of virus mutations relevant to vaccine development may be enhanced. Features of three-dimensional structural information of amino acids may be represented by a graph. By representing such information as a graph, accurate findings relating to amino acids may be obtained, for example, using a machine learning technique suitable for analysis of graph-structured data.

As a technique for comparative analysis of protein structures, for example, a structural alignment method using double dynamic programming has been proposed, which maintains accuracy while achieving time reduction with a simpler method.

### Citation List

### Patent Literature

PTL1: Japanese Laid-open Patent Publication No. 10-185925

### Summary of Invention

### Technical Problem

It is sometimes determined whether amino acids constituting a protein are located within a predetermined distance from each other, thereby determining whether a predetermined relationship exists between amino acids. For example, when a protein such as a virus is represented by a graph, preprocessing is performed in which it is determined whether amino acids constituting the protein are located within a predetermined distance and whether nodes corresponding to a plurality of amino acids are to be connected by an edge. In this preprocessing, a shortest distance between amino acids is calculated for all pairs of amino acids, and it is determined whether nodes corresponding to the amino acids are to be connected by an edge. In general, calculation of the shortest distance between amino acids involves exhaustive calculation of distances between atoms constituting the amino acids. Therefore, when the shortest distance is calculated for all combinations of amino acids, the number of processing targets becomes enormous, and the calculation time increases.

In one aspect, an object of the present disclosure is to enable efficient determination of whether a predetermined relationship exists between amino acids.

### Solution to Problem

In one aspect, there is provided an information processing program that causes a computer to execute the following processing.

The computer executes an information processing program that determines whether a predetermined relationship exists between a plurality of amino acids included in a protein and each composed of a plurality of atoms. The computer calculates, for each of a plurality of amino acid pairs each being a combination of two amino acids among the plurality of amino acids, a distance between the combined amino acids; determines, in response to the distance being equal to or greater than a threshold, that the predetermined relationship does not exist between the combined amino acids; and calculates, in response to the distance being less than the threshold, an inter-atomic distance between an atom constituting one amino acid of the combined amino acids and an atom constituting another amino acid of the combined amino acids, and determines, based on the inter-atomic distance, whether the predetermined relationship exists between the combined amino acids.

### Advantageous Effects of Invention

According to one aspect, it is possible to efficiently determine whether a predetermined relationship exists between amino acids.

The above and other objects, features, and advantages of the present disclosure will become apparent from the following description made in conjunction with the accompanying drawings illustrating preferred embodiments.

### Brief Description of Drawings

[FIG. 1] FIG. 1 illustrates an example of an information processing method according to a first embodiment.
[FIG. 2] FIG. 2 illustrates an example of a system configuration according to a second embodiment.
[FIG. 3] FIG. 3 illustrates an example of hardware of a server.
[FIG. 4] FIG. 4 is a block diagram illustrating an example of a protein analysis processing function using a graph indicating relationships between amino acids.
[FIG. 5] FIG. 5 illustrates an example of a method for generating graphs indicating a three-dimensional structure of a protein.
[FIG. 6] FIG. 6 illustrates an example of a definition of distance between amino acids.
[FIG. 7] FIG. 7 illustrates an example of a distribution state of atoms constituting amino acids.
[FIG. 8] FIG. 8 is a block diagram illustrating an example of functions for graph generation.
[FIG. 9] FIG. 9 illustrates an example of amino acid atom three-dimensional coordinate information.
[FIG. 10] FIG. 10 illustrates an example of amino acid atom list information.
[FIG. 11] FIG. 11 is a flowchart illustrating an example of a procedure of graph generation processing.
[FIG. 12] FIG. 12 illustrates an example of an applicability determination of exclusion for edge definition pair candidates, based on locality information.
[FIG. 13] FIG. 13 is a flowchart illustrating an example of a procedure of distribution locality calculation processing.
[FIG. 14] FIG. 14 illustrates an example of amino acid distribution knowledge information.
[FIG. 15] FIG. 15 illustrates an example of a maximum distribution length in a case where a centroid of an amino acid is used as a representative coordinate.
[FIG. 16] FIG. 16 is a flowchart illustrating an example of a procedure of amino acid distribution knowledge information creation processing.
[FIG. 17] FIG. 17 is a flowchart illustrating an example of a procedure of representative coordinate calculation processing for amino acids.
[FIG. 18] FIG. 18 is a flowchart illustrating an example of a procedure of maximum distribution length calculation processing for amino acids.
[FIG. 19] FIG. 19 illustrates an example of edge definition pair candidates.
[FIG. 20] FIG. 20 illustrates a relationship between an edge threshold and an inter-residue distance.
[FIG. 21] FIG. 21 illustrates an example of exclusion determination processing.
[FIG. 22] FIG. 22 illustrates an example of an exclusion target edge definition pair candidate group and a retention target edge definition pair candidate group.
[FIG. 23] FIG. 23 is a flowchart illustrating an example of a procedure of exclusion determination processing for edge definition pair candidates.
[FIG. 24] FIG. 24 illustrates an example of processing for determining whether an edge connection is established based on an inter-residue distance.
[FIG. 25] FIG. 25 illustrates an example of an inter-residue distance between amino acids.
[FIG. 26] FIG. 26 illustrates an example of graph information output by edge generation processing.
[FIG. 27] FIG. 27 is a flowchart illustrating an example of a procedure of edge generation processing.
[FIG. 28] FIG. 28 illustrates an example of a graph.
[FIG. 29] FIG. 29 illustrates an example of a maximum distribution length when coordinates of a predetermined atom are used as a representative coordinate.

### Description of Embodiments

Embodiments will be described below with reference to the drawings. A plurality of embodiments may be implemented in combination within a range without inconsistency.

### [First Embodiment]

A first embodiment relates to an information processing method for efficiently determining whether a predetermined relationship exists between amino acids in a protein.

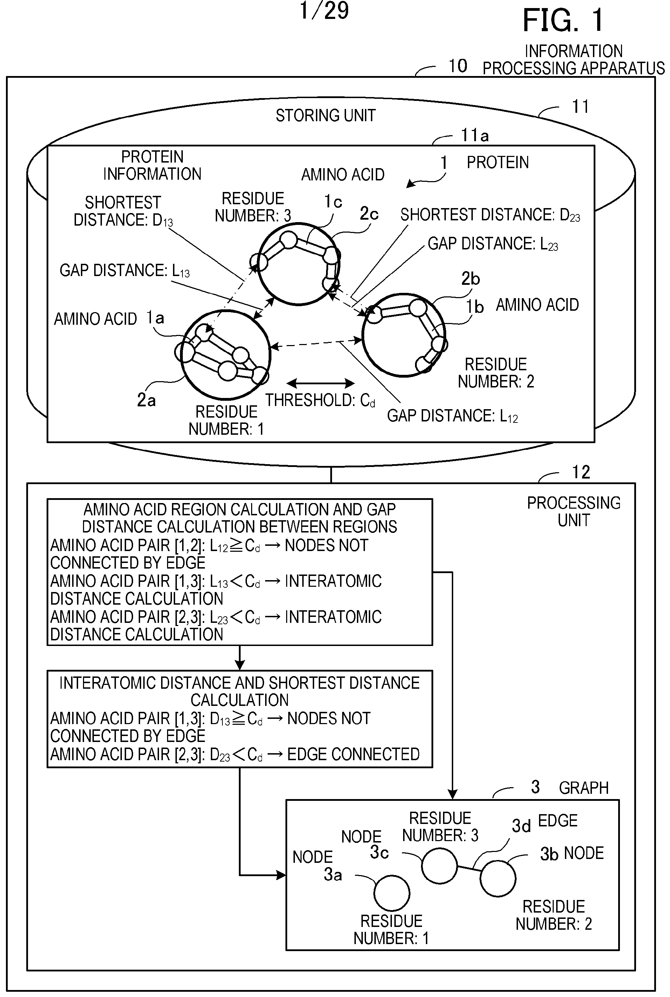

FIG. 1 illustrates an example of an information processing method according to the first embodiment. FIG. 1 illustrates an information processing apparatus 10 for implementing the information processing method according to the first embodiment. The information processing apparatus 10 may implement an information processing method for efficiently determining whether a predetermined relationship exists between amino acids in a protein, for example, by executing a predetermined information processing program. For example, the information processing apparatus 10 may efficiently determine whether nodes corresponding to amino acids in a graph representing a protein are to be connected by an edge. Hereinafter, an example relating to determination of whether nodes corresponding to amino acids in a graph representing a protein are to be connected by an edge will be described. The technology described in the first embodiment may be applied not only to determination of whether nodes in a graph are to be connected by an edge, as long as the technology relates to determination of whether a predetermined relationship exists between amino acids.

The information processing apparatus 10 includes a storing unit 11 and a processing unit 12. The storing unit 11 is, for example, a memory or a storage device included in the information processing apparatus 10. The processing unit 12 is, for example, a processor or an arithmetic circuit included in the information processing apparatus 10.

The storing unit 11 stores protein information 11a indicating a plurality of amino acids 1a to 1c included in a protein 1 and positions of atoms constituting the plurality of amino acids 1a to 1c.

The processing unit 12 calculates a distance between combined amino acids for each of a plurality of amino acid pairs each being a combination of two amino acids among the plurality of amino acids 1a to 1c. When the distance between the combined amino acids is equal to or greater than a threshold, the processing unit 12 determines that no predetermined relationship exists between the combined amino acids. When the distance between the combined amino acids is less than the threshold, the processing unit 12 calculates interatomic distances between atoms constituting one amino acid and atoms constituting the other amino acid in the combined amino acids. Thereafter, the processing unit 12 determines whether the predetermined relationship exists between the combined amino acids based on the interatomic distances.

The processing unit 12 determines whether the nodes 3a to 3c in a graph 3 corresponding to the plurality of amino acids 1a to 1c are to be connected by an edge. An edge 3d represents a feature of the protein 1, and nodes corresponding to two amino acids having a predetermined relationship are connected by an edge, for example. Whether nodes are connected by an edge is determined depending on whether a distance between amino acids is less than a threshold.

That is, for amino acid pairs in which a gap distance between amino acids is equal to or greater than the threshold, the processing unit 12 may determine that no predetermined relationship exists between the combined amino acids without performing interatomic distances. Therefore, compared with a case where interatomic distances are exhaustively calculated for all amino acid pairs to determine whether nodes are to be connected by an edge, the amount of calculation may be reduced.

The distance between amino acids may be a distance between positions (representative coordinates) of the amino acids. As a representative coordinate, for example, a centroid of an amino acid or position coordinates of a predetermined atom may be used. The distance between amino acids may also be a distance between positions within regions (first regions) 2a to 2c described later.

The processing unit 12 calculates the regions (first regions) 2a to 2c including atoms constituting each of the plurality of amino acids 1a to 1c based on the protein information 11a. The protein information 11a is information relating to a protein, including information relating to the plurality of amino acids 1a to 1c and information relating to positions of atoms constituting the plurality of amino acids 1a to 1c.

The processing unit 12 calculates a gap distance between the regions 2a to 2c of combined amino acids for each of a plurality of amino acid pairs generated by combining two amino acids among the plurality of amino acids 1a to 1c. For example, for an amino acid pair of amino acids 1a and 1b, a gap distance between the regions 2a and 2b of the respective combined amino acids 1a and 1b is "L₁₂". For an amino acid pair of amino acids 1a and 1c, a gap distance between the regions 2a and 2c of the respective combined amino acids 1a and 1c is "L₁₃". For an amino acid pair of amino acids 1b and 1c, a gap distance between the regions 2b and 2c of the respective combined amino acids 1b and 1c is "L₂₃".

The processing unit 12 determines whether the nodes 3a to 3c in the graph 3 corresponding to the plurality of amino acids 1a to 1c are to be connected by an edge. The edge 3d represents a feature of the protein 1, and nodes corresponding to two amino acids having a predetermined relationship are connected by an edge. Whether nodes are connected by an edge is determined depending on whether the distance between amino acids is less than a threshold "C_{d}".

For example, the processing unit 12 determines not to connect, by an edge, nodes corresponding to amino acids constituting an amino acid pair (first amino acid pair) in which the gap distance between the regions 2a to 2c is equal to or greater than the threshold "C_{d}".

The processing unit 12 calculates a shortest distance for an amino acid pair (second amino acid pair) in which the gap distance between the regions 2a to 2c is less than the threshold "C_{d}". For example, the processing unit 12 calculates interatomic distances between each atom constituting the first amino acid and each atom constituting the second amino acid, based on positions of atoms constituting the first amino acid and the second amino acid that constitute the second amino acid pair. Thereafter, the processing unit 12 calculates a shortest distance between the first amino acid and the second amino acid based on calculation results of interatomic distances between the first amino acid and the second amino acid. For example, a minimum value of the interatomic distances is the shortest distance between the first amino acid and the second amino acid.

When the shortest distance is equal to or greater than the threshold "C_{d}", the processing unit 12 determines not to connect, by an edge, a first node corresponding to the first amino acid and a second node corresponding to the second amino acid in the graph 3. When the shortest distance is less than the threshold "C_{d}", the processing unit 12 determines to connect the first node and the second node with an edge in the graph 3.

For example, the gap distance "L₁₂" of an amino acid pair of the amino acids 1a and 1b is equal to or greater than the threshold "C_{d}". Therefore, it is determined that the nodes 3a and 3b respectively corresponding to the amino acids 1a and 1b are not connected by an edge.

The gap distance "L₁₃" of an amino acid pair of the amino acids 1a and 1c is less than the threshold "C_{d}". Accordingly, for the amino acid pair of the amino acids 1a and 1c, interatomic distances are exhaustively calculated between atoms constituting the amino acid 1a and atoms constituting the amino acid 1c, and the shortest interatomic distance becomes a shortest distance "D₁₃" between the amino acids 1a and 1c. The shortest distance "D₁₃" between the amino acids 1a and 1c is equal to or greater than the threshold "C_{d}". Therefore, it is determined that the nodes 3a and 3c respectively corresponding to the amino acids 1a and 1c are not connected by an edge.

The gap distance "L₂₃" of an amino acid pair of the amino acids 1b and 1c is less than the threshold "C_{d}". Accordingly, for the amino acid pair of the amino acids 1b and 1c, interatomic distances are exhaustively calculated between atoms constituting the amino acid 1b and atoms constituting the amino acid 1c, and the shortest interatomic distance becomes a shortest distance "D₂₃" between the amino acids 1b and 1c. The shortest distance "D₂₃" between the amino acids 1b and 1c is less than the threshold "C_{d}". Therefore, it is determined that the nodes 3b and 3c respectively corresponding to the amino acids 1b and 1c are connected by the edge 3d.

The processing unit 12 generates the graph 3 in which nodes are connected by edges as determined. By generating the graph 3 in this manner, the processing unit 12 is capable of efficiently determining whether the nodes 3a to 3c included in the graph 3 are to be connected by an edge. That is, for amino acid pairs in which the gap distance between the regions is equal to or greater than the threshold "C_{d}", the processing unit 12 determines not to connect the nodes by an edge without performing interatomic distances. Therefore, compared with a case where interatomic distances are exhaustively calculated for all amino acid pairs to determine whether nodes are to be connected by an edge, the amount of calculation may be reduced.

The processing unit 12 calculates representative coordinates of an amino acid to be calculated based on positions of atoms constituting the amino acid to be calculated, and determines, for amino acid pairs in which a gap distance between the representative coordinates is equal to or greater than a threshold, not to connect the nodes by an edge without performing interatomic distances. Therefore, compared with a case where interatomic distances are exhaustively calculated for all amino acid pairs to determine whether nodes are to be connected by an edge, the amount of calculation may be reduced.

The processing unit 12 calculates, for each amino acid, for example, spherical regions 2a to 2c as the regions 2a to 2c including atoms constituting the amino acid. In that case, the processing unit 12 calculates representative coordinates of the amino acid to be calculated based on positions of atoms constituting the amino acid to be calculated. The representative coordinates are, for example, coordinates of a centroid of the amino acid to be calculated. The processing unit 12 then calculates a spherical region including atoms constituting the amino acid to be calculated with the representative coordinates as the center.

By using the spherical regions 2a to 2c, a gap distance between regions of two amino acids constituting an amino acid pair may be obtained by simple calculation. As a result, the processing is made more efficient.

When calculating the spherical regions 2a to 2c, the processing unit 12 determines radii of the regions 2a to 2c based on distances from the representative coordinates to atoms constituting the amino acid to be calculated. For example, the processing unit 12 sets a maximum value among distances from the representative coordinates to the atoms constituting the amino acid to be calculated, as the radius of the regions 2a to 2c. Accordingly, the processing unit 12 generates the regions 2a to 2c having the smallest radius and including all atoms constituting the amino acid to be calculated. When the radius of the regions 2a to 2c is smaller, the gap distance between the regions becomes larger, and the number of amino acid pairs for which it is determined not to connect nodes by an edge without calculating interatomic distances increases. As a result, the processing is made more efficient.

The processing unit 12 may calculate a gap distance by sampling amino acid pairs, calculate a ratio of amino acid pairs having a possibility that a shortest distance does not exceed the threshold "C_{d}", and calculate interatomic distances for all amino acid pairs when the ratio is greater than a predetermined value.

For example, the processing unit 12 extracts some of a plurality of amino acid pairs as amino acid pairs to be sampled. Next, the processing unit 12 calculates, for an amino acid to be sampled included in any one of the amino acid pairs to be sampled, a region (second region) including atoms constituting the amino acid. A calculation method of the second region is the same as that of the first region.

The processing unit 12 calculates, for each of the amino acid pairs to be sampled, a gap distance between the second regions of the combined amino acids. The processing unit 12 then calculates, among the amino acid pairs to be sampled, a ratio of amino acid pairs in which the gap distance between the second regions is less than the threshold "C_{d}". This ratio is a ratio of amino acid pairs for which a possibility remains that a shortest distance between amino acids is less than the threshold "C_{d}".

When the ratio exceeds a predetermined value, the processing unit 12 suppresses execution of predetermined processing. The processing to be suppressed includes processing for calculating the first regions, processing for calculating a gap distance between the first regions, and processing for determining not to connect, by an edge, nodes corresponding to amino acids constituting a first amino acid pair in which the gap distance between the first regions is equal to or greater than the threshold.

When the ratio exceeds the predetermined value, the processing unit 12 calculates, with each of the plurality of amino acid pairs as a calculation target, a shortest distance between a third amino acid and a fourth amino acid constituting the amino acid pair to be calculated. For example, the processing unit 12 calculates interatomic distances between atoms constituting the third amino acid and atoms constituting the fourth amino acid based on positions of atoms constituting the third amino acid and the fourth amino acid. The processing unit 12 then calculates the shortest distance between the third amino acid and the fourth amino acid based on calculation results of the interatomic distances between the third amino acid and the fourth amino acid.

When the shortest distance between the third amino acid and the fourth amino acid is equal to or greater than the threshold, the processing unit 12 determines not to connect, by an edge, a third node corresponding to the third amino acid and a fourth node corresponding to the fourth amino acid in the graph 3. When the shortest distance between the third amino acid and the fourth amino acid is less than the threshold, the processing unit 12 determines to connect the third node and the fourth node with an edge in the graph 3.

When it is found, by sampling, that the ratio of amino acid pairs having a possibility that a shortest distance does not exceed the threshold "C_{d}" is greater than a predetermined value, the number of amino acid pairs for which it is determined, based on calculation results of gap distances between regions of amino acids, not to connect nodes by an edge without calculating interatomic distances becomes small. Therefore, in such a case, the processing unit 12 performs the shortest distance based on interatomic distances for all amino acid pairs, and suppresses calculation of gap distances between regions. Accordingly, useless calculation of gap distances between regions is suppressed, and processing efficiency may be improved.

### [Second Embodiment]

A second embodiment is a system capable of efficiently determining whether to connect nodes by an edge, when a chemical action acting between amino acids constituting a protein is regarded as a relationship, and the relationship is represented by an edge between nodes representing amino acids.

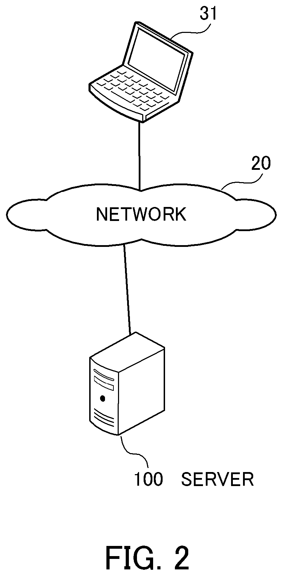

FIG. 2 illustrates an example of a system configuration according to the second embodiment. A server 100 that performs structural analysis of a protein using graph-based artificial intelligence (AI) is connected to a terminal apparatus 31 via a network 20. The terminal apparatus 31 is a computer used by a user. The server 100 analyzes, for example, a structure of a virus protein in response to a request from the terminal apparatus 31, and performs, for example, identification of a virus type.

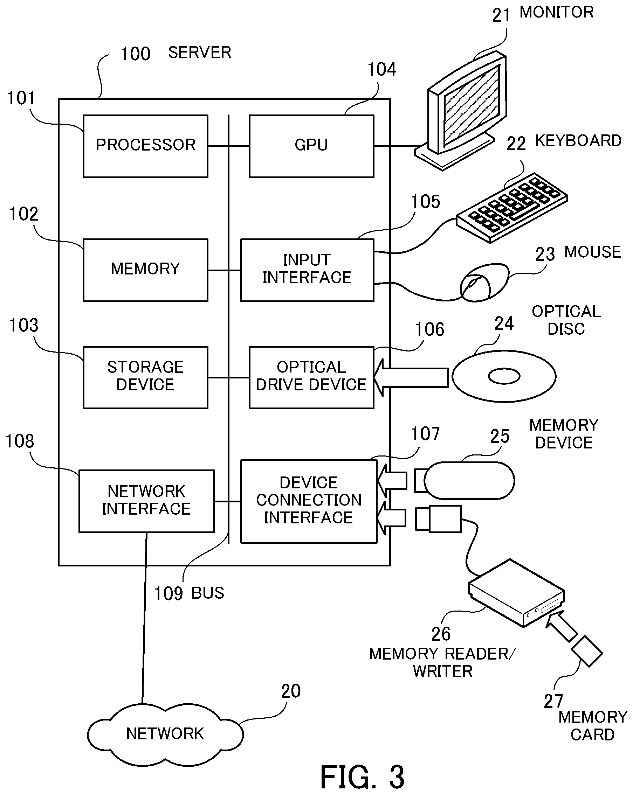

FIG. 3 illustrates an example of hardware of the server. The server 100 is entirely controlled by a processor 101. A memory 102 and a plurality of peripheral devices are connected to the processor 101 via a bus 109. The processor 101 may be a multiprocessor. The processor 101 is, for example, a central processing unit (CPU), a micro processing unit (MPU), or a digital signal processor (DSP). At least a part of functions realized by the processor 101 executing a program may be realized by an electronic circuit such as an application specific integrated circuit (ASIC) or a programmable logic device (PLD).

The memory 102 is used as a main storage device of the server 100. At least a part of an operating system (OS) program and an application program to be executed by the processor 101 is temporarily stored in the memory 102. Various data used for processing by the processor 101 is also stored in the memory 102. As the memory 102, for example, a volatile semiconductor storage device such as a random access memory (RAM) is used.

As peripheral devices connected to the bus 109, the peripheral devices include a storage device 103, a graphics processing unit (GPU) 104, an input interface 105, an optical drive device 106, a device connection interface 107, and a network interface 108.

The storage device 103 electrically or magnetically writes data to, and reads data from, a built-in recording medium. The storage device 103 is used as an auxiliary storage device of the server 100. An OS program, an application program, and various data are stored in the storage device 103. As the storage device 103, for example, a hard disk drive (HDD) or a solid state drive (SSD) may be used.

The GPU 104 is an arithmetic device that performs image processing. The GPU 104 is an example of a graphic controller. A monitor 21 is connected to the GPU 104. The GPU 104 displays an image on a screen of the monitor 21 in accordance with an instruction from the processor 101. Examples of the monitor 21 include a display device using an organic electro luminescence (EL) and a liquid crystal display device.

A keyboard 22 and a mouse 23 are connected to the input interface 105. The input interface 105 transmits signals sent from the keyboard 22 and the mouse 23 to the processor 101. The mouse 23 is an example of a pointing device, and another pointing device may be used. Examples of the other pointing device include a touch panel, a tablet, a touch pad, and a trackball.

The optical drive device 106 reads data recorded on an optical disc 24 or writes data to the optical disc 24, using laser light or the like. The optical disc 24 is a portable recording medium on which data is recorded so as to be readable by reflection of light. Examples of the optical disc 24 include a digital versatile disc (DVD), a DVD-RAM, a compact disc read only memory (CD-ROM), and a CD-recordable/rewriteable (CD-R/RW).

The device connection interface 107 is a communication interface for connecting a peripheral device to the server 100. For example, a memory device 25 and a memory reader/writer 26 may be connected to the device connection interface 107. The memory device 25 is a recording medium equipped with a communication function with the device connection interface 107. The memory reader/writer 26 is a device that writes data to a memory card 27 or reads data from the memory card 27. The memory card 27 is a card-type recording medium.

The network interface 108 is connected to the network 20. The network interface 108 transmits and receives data to and from another computer or a communication device via the network 20. The network interface 108 is, for example, a wired communication interface connected by a cable to a wired communication device such as a switch or a router. The network interface 108 may also be a wireless communication interface that is communication-connected by radio waves to a wireless communication device such as a base station or an access point.

The server 100 realizes the processing functions of the second embodiment by the hardware as described above. The information processing apparatus 10 described in the first embodiment may also be realized by hardware similar to the server 100 illustrated in FIG. 3.

The server 100 realizes the processing functions of the second embodiment, for example, by executing a program recorded in a computer-readable recording medium. A program describing processing content to be executed by the server 100 may be recorded in various recording media. For example, a program to be executed by the server 100 may be stored in the storage device 103. The processor 101 loads at least a part of the program in the storage device 103 into the memory 102 and executes the program. A program to be executed by the server 100 may also be recorded in a portable recording medium such as the optical disc 24, the memory device 25, or the memory card 27. A program stored in the portable recording medium becomes executable, for example, after being installed in the storage device 103 under control by the processor 101. The processor 101 may also read a program directly from the portable recording medium and execute the program.

The server 100 performs, for example, classification (clustering) of proteins using relationships between amino acids constituting the proteins. In this case, the server 100 classifies proteins using a machine learning model capable of effectively classifying graphs, by generating, for various proteins, graphs indicating relationships between amino acids.

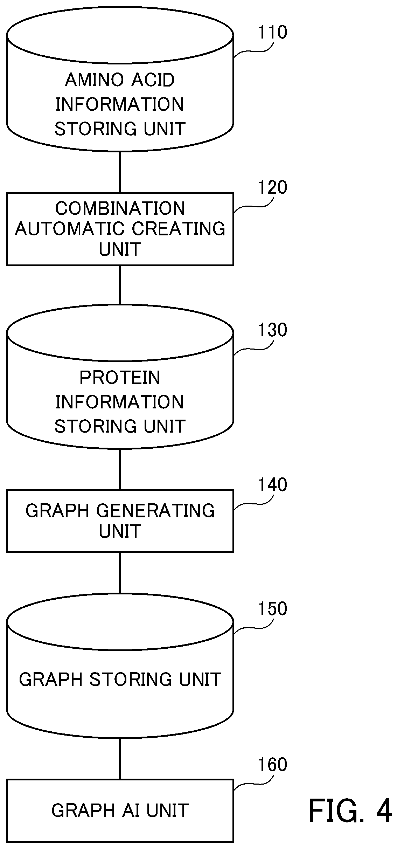

FIG. 4 is a block diagram illustrating an example of a protein analysis processing function using a graph indicating relationships between amino acids. The server 100 includes an amino acid information storing unit 110, a combination automatic creating unit 120, a protein information storing unit 130, a graph generating unit 140, a graph storing unit 150, and a graph AI unit 160.

The amino acid information storing unit 110 stores, for example, knowledge information of a knowledge graph region for a plurality of amino acids. In the knowledge graph region knowledge information, information such as structures of amino acids is represented in a knowledge graph.

The combination automatic creating unit 120 automatically generates combinations of amino acids constituting a protein based on information indicated in the amino acid information storing unit 110. The combination automatic creating unit 120 stores, in the protein information storing unit 130, information indicating a protein represented by the created combinations of amino acids.

The protein information storing unit 130 stores information such as structures of proteins. The information of the proteins includes, for example, information such as electric charge amounts, hydrophilicity, hydrophobicity, and molecular weights of constituting amino acids. The information of the proteins also includes information such as peptide bonds between amino acids and van der Waals forces.

The graph generating unit 140 creates a graph representing features such as a three-dimensional structure of a protein based on the information of the protein indicated in the protein information storing unit 130. For example, when representing the three-dimensional structure of the protein as a graph, the graph generating unit 140 measures distances between amino acids and determines whether edges are connected based on a threshold. The graph generating unit 140 stores the generated graph in the graph storing unit 150.

The graph AI unit 160 estimates, for example, a class to which a protein belongs, based on graphs representing features of proteins stored in the graph storing unit 150 by using, for example, the generated graph.

When a graph indicating a three-dimensional structure of a protein is analyzed using graph AI in this manner, a type of virus strain is accurately discriminated, for example. When the type of the virus strain is discriminated, analysis of virus mutations is improved.

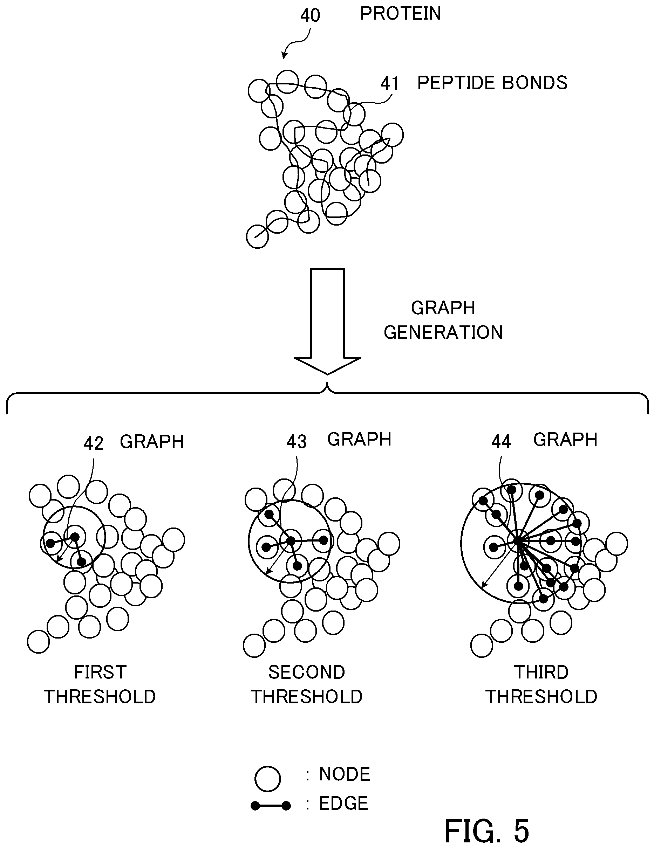

FIG. 5 illustrates an example of a method for generating graphs indicating a three-dimensional structure of a protein. A protein 40 has a three-dimensional structure formed by folding a single sequence formed by peptide bonds 41 between amino acids. When amino acids regarded as nodes (indicated by circles in the drawing) become close to each other due to such folding, chemical actions act between those amino acids. Therefore, graphs 42 to 44 reflecting actions acting between amino acids are generated by configuring edges (lines connecting nodes) while regarding actions between amino acids as relationships.

However, a distance between amino acids at which a chemical action acts is not clearly known. Therefore, a threshold relating to a distance used for determining whether edges are connected is arbitrarily set according to a hypothesis regarding at what distance an action acting between amino acids is regarded as effective. In other words, the threshold is not predetermined and is changed according to requirements of a usage situation, and the graphs 42 to 44 are generated each time.

Since the graphs 42 to 44 are generated by creating edges based on distances between amino acids, whether nodes are connected by edges depends on how the distances between amino acids are defined.

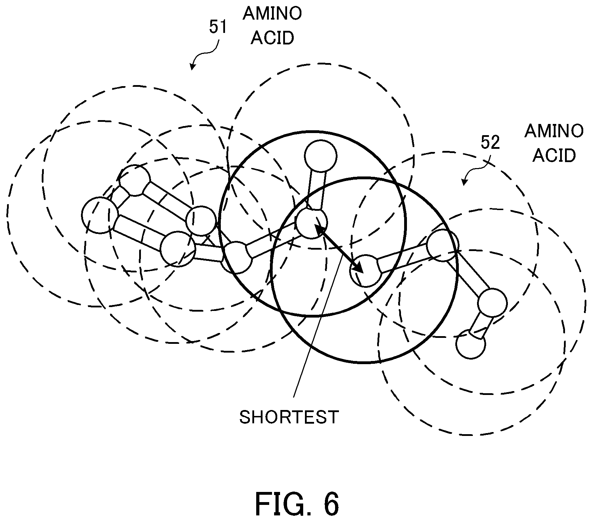

FIG. 6 illustrates an example of a definition of distance between amino acids. For example, a shortest value of distances between atoms included in two amino acids 51 and 52 is used as the distance between amino acids. Generation of a graph based on such distances between amino acids is performed, for example, according to requirements in a proof of concept (PoC).

When the shortest value of distances between atoms is used as the distance between amino acids, distances between atoms constituting the amino acids are generally calculated exhaustively in order to generate the graph. In other words, which pair of atoms forms the shortest distance is not known until distances between all atoms are calculated and compared. Therefore, when the distance between amino acids (inter-residue distance) is defined as the shortest distance, distances between all atoms are calculated unless any improvement is introduced.

For example, a total number of atoms in a protein is defined as "M_{All} = Σᵢ₌₁^{N}Mᵢ". When all atom pairs are exhaustively calculated, the computational complexity is represented as O(M_{All}²) in order notation. In an actual problem, distance calculations on the order of approximately one hundred million divided by two occur.

In a case such as discrimination of virus species, a protein includes ten thousand or more atoms. In that case, distance calculations on the order of approximately ten thousand multiplied by ten thousand are performed. As a result, the number of processing targets becomes enormous, and calculation takes time. When a large number of protein samples (for example, one hundred or more samples) are present, several hours may be needed per sample, and determining graphs for all samples may take several weeks.

Here, atoms constituting amino acids are not distributed randomly in space but are distributed with bias. Therefore, the graph generating unit 140 uses knowledge regarding distributions of atoms constituting amino acids to reduce the amount of computation during graph generation.

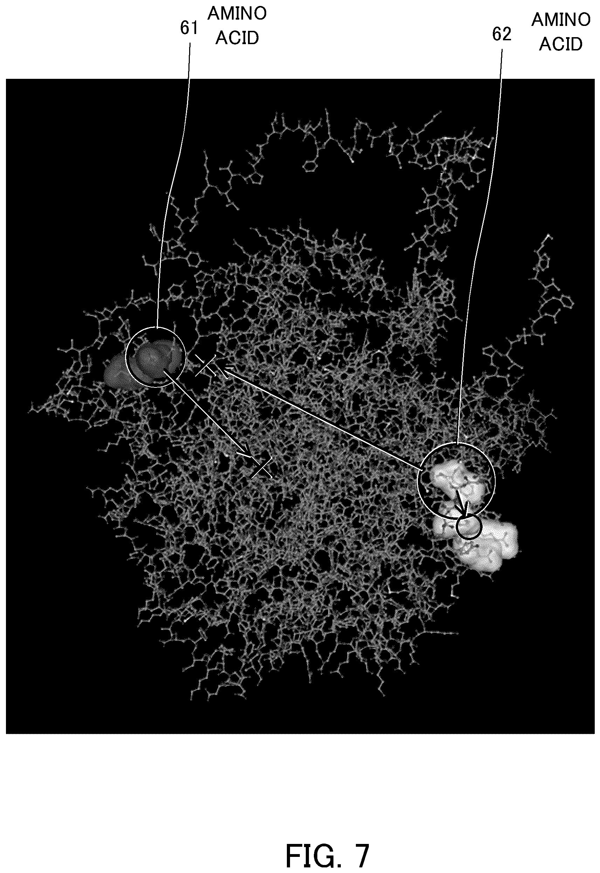

FIG. 7 illustrates an example of a distribution state of atoms constituting amino acids. In FIG. 7, some amino acids 61 and 62 constituting a protein 60 are indicated by closed surfaces enclosing atoms constituting the amino acids 61 and 62.

As illustrated in FIG. 7, atoms constituting each of the amino acids 61 and 62 exist while being locally clustered. That is, atoms constituting each of the amino acids 61 and 62 do not exist at discontinuously distant locations but exist within a certain distance. Therefore, in a structure of a protein, when two amino acids are randomly selected, the distribution of atoms becomes such that the ratio of cases in which atoms of one amino acid exist near another amino acid becomes very small. Moreover, for one amino acid, the number of other amino acids with which interactions occur is at most about ten. Therefore, for the remaining approximately 3,800 amino acids, a reduction in computational load is achieved if interatomic distances are not calculated.

Accordingly, the graph generating unit 140 efficiently performs graph generation using protein three-dimensional coordinate information, knowledge information regarding amino acid constituent distributions, and distribution locality information.

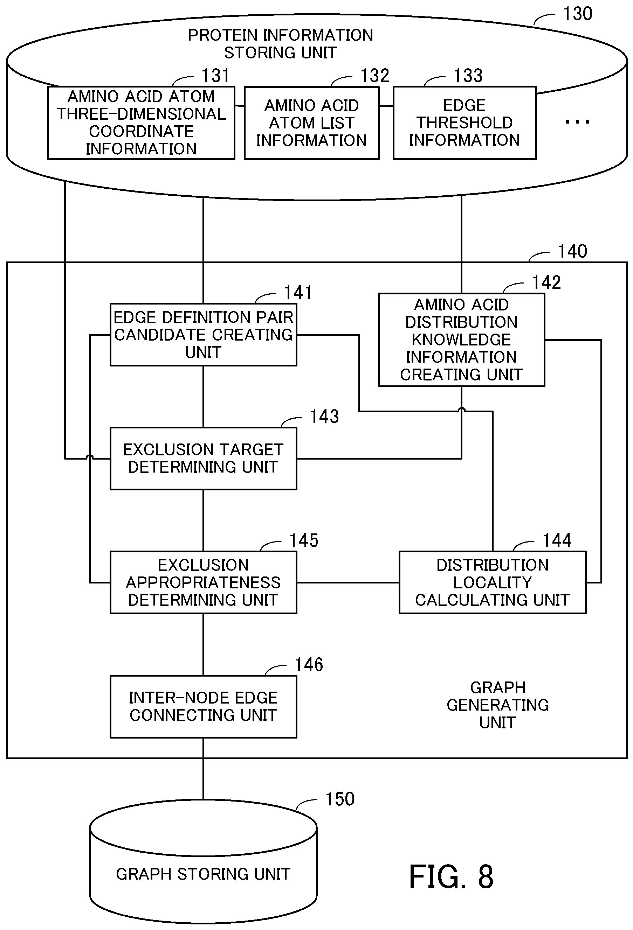

FIG. 8 is a block diagram illustrating an example of functions for graph generation. The graph generating unit 140 generates a graph based on information of a protein stored in the protein information storing unit 130. Examples of the information of the protein include amino acid atom three-dimensional coordinate information 131, amino acid atom list information 132, and edge threshold information 133.

The amino acid atom three-dimensional coordinate information 131 indicates coordinates of atoms included in amino acids in a three-dimensional space. The amino acid atom list information 132 indicates atoms constituting each amino acid. The edge threshold information 133 indicates threshold information of distances between amino acids (edge threshold) used for determining whether edges are connected between nodes when generating a graph.

The graph generating unit 140 includes an edge definition pair candidate creating unit 141, an amino acid distribution knowledge information creating unit 142, an exclusion target determining unit 143, a distribution locality calculating unit 144, an exclusion appropriateness determining unit 145, and an inter-node edge connecting unit 146.

The edge definition pair candidate creating unit 141 creates candidates of amino acid pairs defining edges in a graph (edge definition pair candidates). For example, the edge definition pair candidate creating unit 141 sets, as edge definition pair candidates, all combinations generated by selecting two amino acids from amino acids included in a protein.

The amino acid distribution knowledge information creating unit 142 generates amino acid distribution knowledge information indicating distribution states of atoms constituting each amino acid. For example, the amino acid distribution knowledge information creating unit 142 calculates representative coordinates indicating positions of amino acids and sets, as amino acid distribution knowledge information, distances from the representative coordinates to the farthest atoms.

The exclusion target determining unit 143 determines, for each edge definition pair candidate, whether the edge definition pair candidate is excluded from calculation targets of inter-residue distances. For example, based on the amino acid distribution knowledge information, the exclusion target determining unit 143 determines, as exclusion targets, edge definition pair candidates for which inter-residue distances are clearly equal to or greater than the edge threshold.

The distribution locality calculating unit 144 calculates locality information indicating locality of amino acid distributions based on the amino acid distribution knowledge information. The locality of distributions is represented, for example, by a ratio of amino acid definition pair candidates having distances that may fail to exceed the edge threshold (that is, amino acid definition pair candidates having short distances). The higher the ratio of amino acid definition pair candidates having short distances, the lower the locality becomes.

The exclusion appropriateness determining unit 145 determines, based on the locality information of distributions, whether edge definition pair candidates determined as exclusion targets are excluded from calculation targets of inter-residue distances. For example, when the locality of distributions is higher than a predetermined value, the exclusion appropriateness determining unit 145 determines that exclusion target edge definition pair candidates are excluded from calculation targets of inter-residue distances.

The inter-node edge connecting unit 146 calculates inter-residue distances between edge definition pair candidates and generates edges connecting nodes corresponding to edge definition pair candidates for which the inter-residue distances are equal to or less than the edge threshold. At this time, when it is determined that exclusion target edge definition pair candidates are excluded from calculation targets, the inter-node edge connecting unit 146 determines not to calculate inter-residue distances and not to define an edge for the exclusion target edge definition pair candidates. The inter-node edge connecting unit 146 stores information on the generated edges in the graph storing unit 150 as graph information.

Functions of respective elements illustrated in FIG. 8 are realized, for example, by causing the processor 101 to execute program modules corresponding to the respective elements.

Next, data used for graph generation will be specifically described.

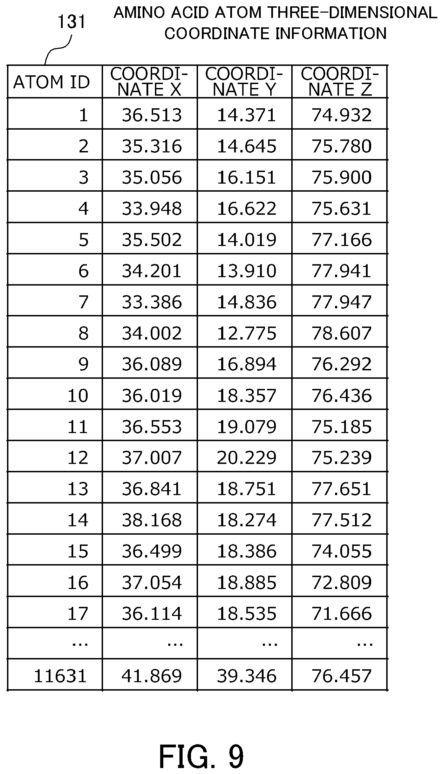

FIG. 9 illustrates an example of the amino acid atom three-dimensional coordinate information. In the amino acid atom three-dimensional coordinate information 131, an X-axis coordinate (coordinate X), a Y-axis coordinate (coordinate Y), and a Z-axis coordinate (coordinate Z) are set in association with an atom ID identifying each atom included in a protein.

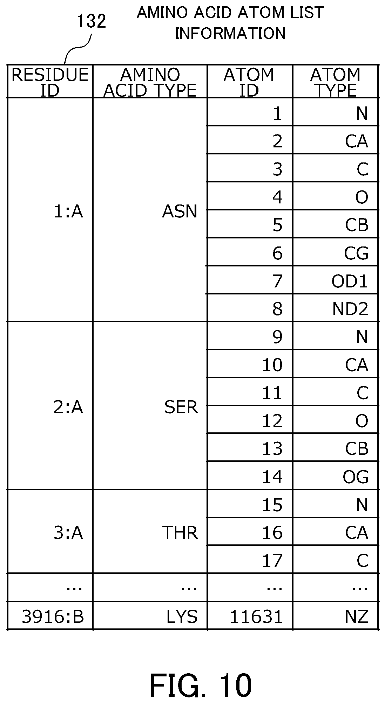

FIG. 10 illustrates an example of the amino acid atom list information. In the amino acid atom list information 132, an amino acid type, an atom ID, and an atom type are set in association with a residue ID identifying an amino acid constituting a protein. A number on the left side of the residue ID is an identification number (residue number) uniquely assigned to each amino acid.

The amino acid type is a name indicating a type of amino acid. The atom ID is an atom ID of an atom constituting an amino acid. The atom type is a type of an atom indicated by the atom ID. In the example illustrated in FIG. 10, atoms having atom IDs "1" to "8" constitute an amino acid (ASN: asparagine) having the residue ID "1:A".

In addition to the amino acid atom three-dimensional coordinate information 131 and the amino acid atom list information 132 illustrated in FIGS. 9 and 10, the protein information storing unit 130 stores edge threshold information 133. The edge threshold information 133 is set to a value by a user requesting analysis.

Next, graph generation processing based on information of a protein will be described in detail.

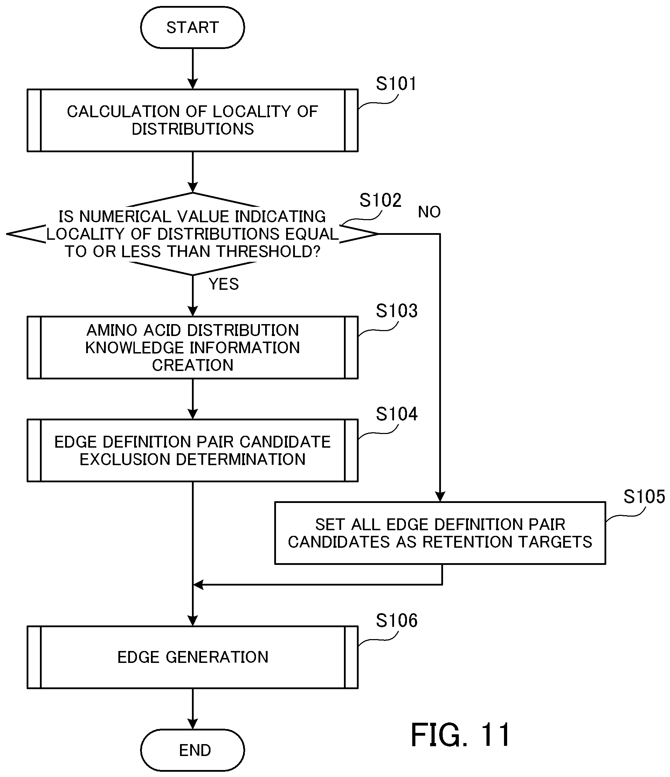

FIG. 11 is a flowchart illustrating an example of a procedure of graph generation processing. Hereinafter, the processing illustrated in FIG. 11 will be described according to step numbers.

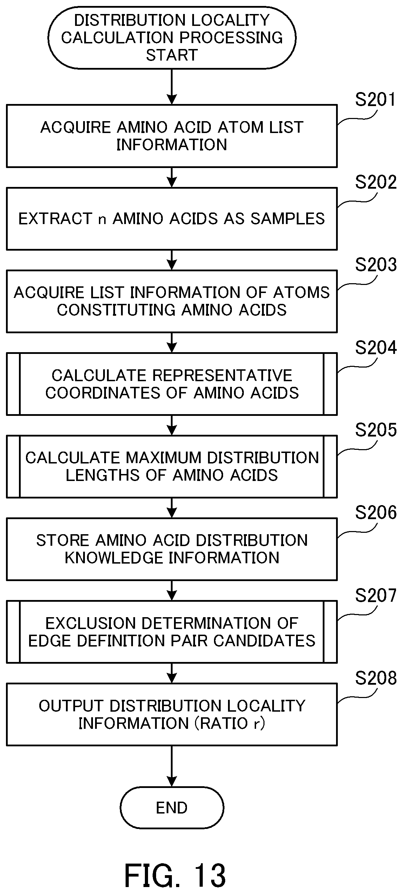

[Step S101] The graph generating unit 140 calculates locality of distributions. The calculation of the locality of the distributions is processing mainly performed by the distribution locality calculating unit 144. Details of the calculation processing of the locality of the distributions will be described later (see FIG. 13).

[Step S102] The exclusion appropriateness determining unit 145 of the graph generating unit 140 determines whether a numerical value indicating the locality of the distributions (a ratio r of amino acid pairs whose distances may fail to exceed the edge threshold) is equal to or less than a threshold. When the numerical value indicating the locality of the distributions is equal to or less than the threshold, the exclusion appropriateness determining unit 145 advances the processing to step S103. When the numerical value indicating the locality of the distributions exceeds the threshold, the exclusion appropriateness determining unit 145 advances the processing to step S105.

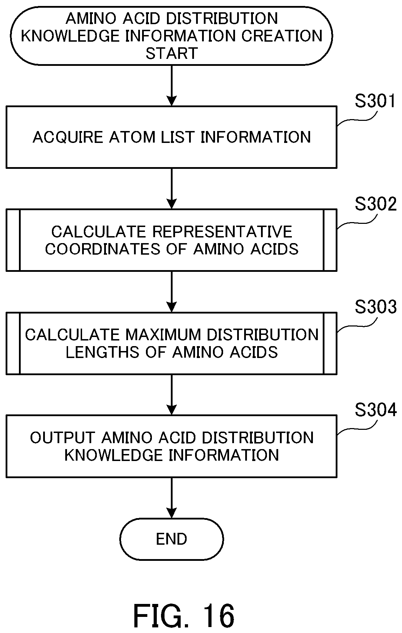

[Step S103] The amino acid distribution knowledge information creating unit 142 of the graph generating unit 140 performs amino acid distribution knowledge information creation processing. Details of the amino acid distribution knowledge information creation processing will be described later (see FIG. 16).

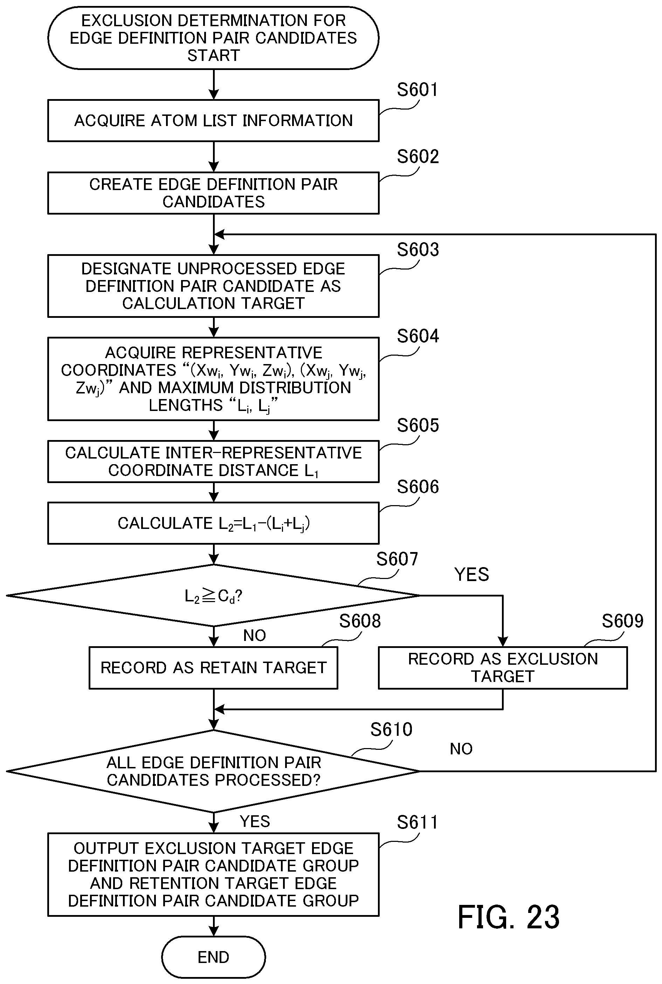

[Step S104] The graph generating unit 140 performs edge definition pair candidate exclusion determination processing. The edge definition pair candidate exclusion determination processing is processing mainly performed by the exclusion target determining unit 143. In the edge definition pair candidate exclusion determination processing, each edge definition pair candidate is classified into an exclusion target edge definition pair candidate excluded from calculation targets of inter-residue distances between amino acids and a retention target edge definition pair candidate included in calculation targets of inter-residue distances between amino acids. Details of the exclusion determination processing of edge definition pair candidates will be described later (see FIG. 23). Thereafter, the graph generating unit 140 advances the processing to step S106.

[Step S105] The graph generating unit 140 sets all edge definition pair candidates as retention target edge definition pair candidates.

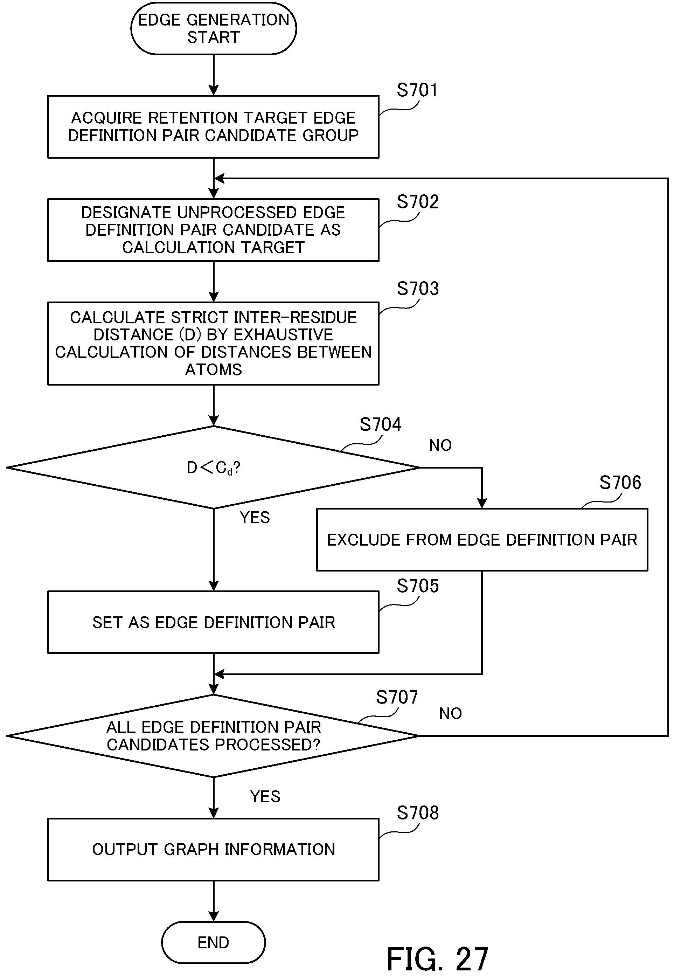

[Step S106] The inter-node edge connecting unit 146 of the graph generating unit 140 generates edges between nodes corresponding to amino acids based on inter-residue distances between amino acids for the retention target edge definition pair candidates. Information on edges connecting the nodes is stored in the graph storing unit 150 as a graph. Details of the edge generation processing will be described later (see FIG. 27).

When the numerical value indicating the locality of the distributions is equal to or less than the threshold (that is, when the locality is high), exclusion target edge definition pair candidates are excluded from calculation targets of inter-residue distances. In this case, inter-residue distances are calculated for edge definition pair candidates that are not excluded, and edges are generated when the inter-residue distances are equal to or less than the edge threshold. On the other hand, when the locality of the distributions exceeds the threshold (that is, when the locality is low), inter-residue distances are calculated for all edge definition pair candidates, and edges are generated when the inter-residue distances are equal to or less than the edge threshold.

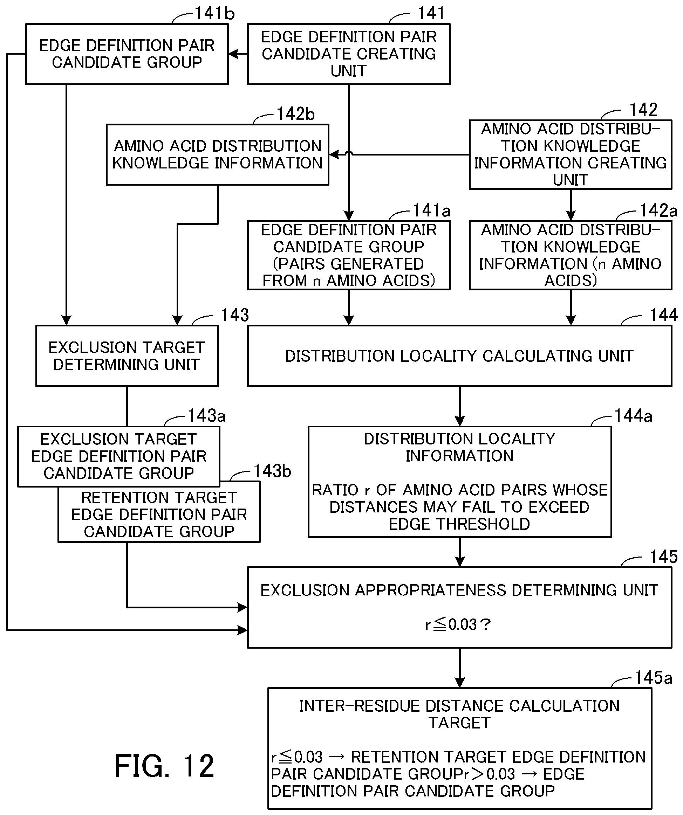

FIG. 12 illustrates an example of an applicability determination of exclusion for edge definition pair candidates, based on locality information. For example, the edge definition pair candidate creating unit 141 generates pairs of two amino acids (edge definition pair candidates) from n amino acids (n being a natural number) selected as samples, and transmits an extracted edge definition pair candidate group 141a to the distribution locality calculating unit 144.

The edge definition pair candidate creating unit 141 also generates an edge definition pair candidate group 141b including all edge definition pair candidates that are generated from all amino acids included in the protein. The edge definition pair candidate creating unit 141 transmits the generated edge definition pair candidate group 141b to the exclusion target determining unit 143 and the exclusion appropriateness determining unit 145.

The amino acid distribution knowledge information creating unit 142 creates amino acid distribution knowledge information 142a for n amino acids selected as samples. The amino acid distribution knowledge information 142a indicates, for each amino acid, a representative coordinate and a distance from the representative coordinate to a farthest atom. The amino acid distribution knowledge information creating unit 142 transmits the generated amino acid distribution knowledge information 142a to the distribution locality calculating unit 144.

The amino acid distribution knowledge information creating unit 142 also creates amino acid distribution knowledge information 142b for all amino acids included in the protein. The amino acid distribution knowledge information creating unit 142 transmits the created amino acid distribution knowledge information 142b to the exclusion target determining unit 143.

The distribution locality calculating unit 144 calculates locality information of distributions based on the amino acid distribution knowledge information 142a. For example, the distribution locality calculating unit 144 estimates, from the edge definition pair candidate group 141a including N edge definition pair candidates, a ratio r of edge definition pair candidates whose distances may fail to exceed the edge threshold. The ratio r is a predicted value of a ratio of edge definition pair candidates belonging to a retention target edge definition pair candidate group 143b among all edge definition pair candidates. The distribution locality calculating unit 144 sets the estimated ratio r as distribution locality information 144a.

Whether an edge definition pair candidate may have an inter-residue distance that does not exceed the edge threshold is determined in the same manner as a determination by the exclusion target determining unit 143 as to whether the edge definition pair candidate is to be retained.

The exclusion appropriateness determining unit 145 determines, based on the distribution locality information 144a, whether it is efficient to calculate inter-residue distances using combinations of pairs of some amino acids instead of combinations of pairs among all amino acids by using knowledge information regarding amino acid constituent distributions. For example, when the ratio r indicated in the distribution locality information 144a is equal to or less than a predetermined threshold (for example, 0.03), the exclusion appropriateness determining unit 145 determines that calculating inter-residue distances using combinations of some amino acids is efficient.

When the ratio r indicated in the distribution locality information 144a exceeds the predetermined threshold, the exclusion appropriateness determining unit 145 determines that it is efficient to calculate inter-residue distances for all edge definition pair candidates capable of being generated. In other words, when the ratio r exceeds the threshold, a ratio of edge definition pair candidates to be excluded becomes small. In such a case, all edge definition pair candidates are transmitted to the inter-residue distance calculation target 145a of the inter-node edge connecting unit 146. Accordingly, execution of a determination process by the exclusion target determining unit 143 for determining edge definition pair candidates to be excluded is omitted.

When the ratio r is equal to or less than the predetermined threshold, the exclusion target determining unit 143 classifies the edge definition pair candidates in the edge definition pair candidate group 141b into either exclusion target edge definition pair candidates or retention target edge definition pair candidates. The exclusion target determining unit 143 transmits an exclusion target edge definition pair candidate group 143a and the retention target edge definition pair candidate group 143b to the exclusion appropriateness determining unit 145. The exclusion appropriateness determining unit 145 transmits the retention target edge definition pair candidate group 143b to the inter-node edge connecting unit 146 as an inter-residue distance calculation target 145a.

Next, a procedure of a distribution locality calculation processing will be described in detail.

FIG. 13 is a flowchart illustrating an example of a procedure of the distribution locality calculation processing. The processing illustrated in FIG. 13 will be described below according to step numbers.

[Step S201] The amino acid distribution knowledge information creating unit 142 acquires the amino acid atom list information 132 from the protein information storing unit 130.

[Step S202] The amino acid distribution knowledge information creating unit 142 extracts amino acid information corresponding to a number n of samples (n being a natural number) from the amino acid atom list information 132.

[Step S203] The amino acid distribution knowledge information creating unit 142 acquires list information of atoms constituting the extracted amino acids.

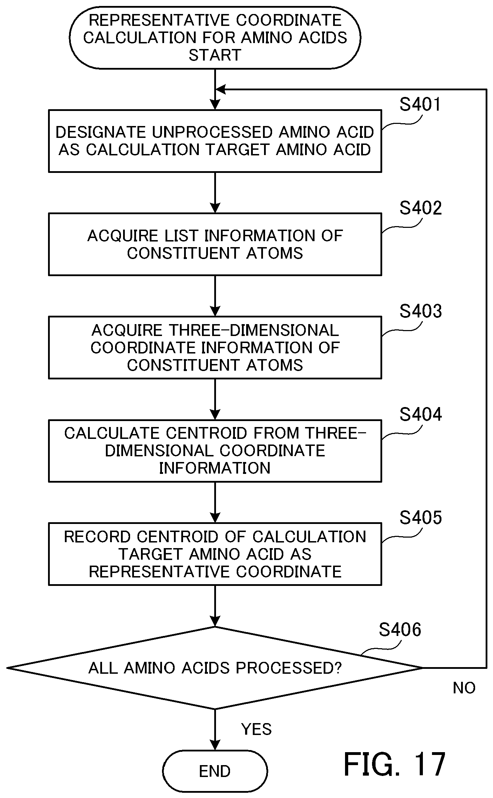

[Step S204] The amino acid distribution knowledge information creating unit 142 calculates a representative coordinate of each of the n amino acids. Details of a representative coordinate calculation processing for amino acids will be described later (see FIG. 17).

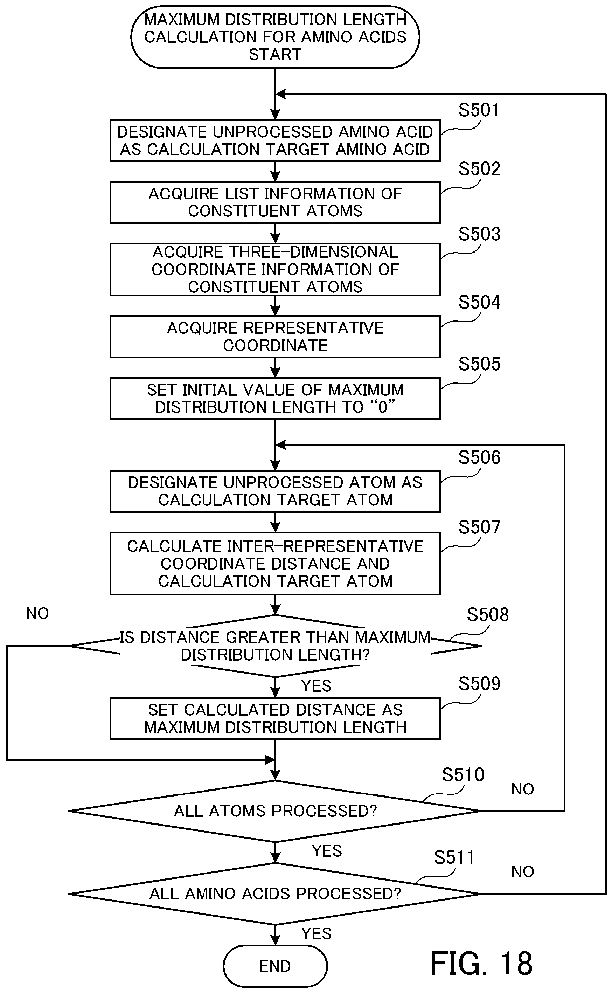

[Step S205] The amino acid distribution knowledge information creating unit 142 calculates maximum distribution lengths of the n amino acids. Details of a maximum distribution length calculation processing for amino acids will be described later (see FIG. 18).

[Step S206] The amino acid distribution knowledge information creating unit 142 stores amino acid distribution knowledge information for the n amino acids in the memory 102 or the storage device 103.

[Step S207] The distribution locality calculating unit 144 performs an exclusion determination on each edge definition pair candidate formed by combining two amino acids among the n amino acids. Details of an exclusion determination processing for edge definition pair candidates will be described later (see FIG. 23).

[Step S208] The distribution locality calculating unit 144 outputs, as distribution locality information, the ratio r of amino acid pairs whose distances may fail to exceed the edge threshold. The ratio r is a ratio of edge definition pair candidates determined to be retained in the exclusion determination processing for edge definition pair candidates among edge definition pair candidates obtained from the sampled amino acids.

In this manner, the distribution locality information is generated. The amino acid distribution knowledge information 142a is used for calculation of distribution locality information.

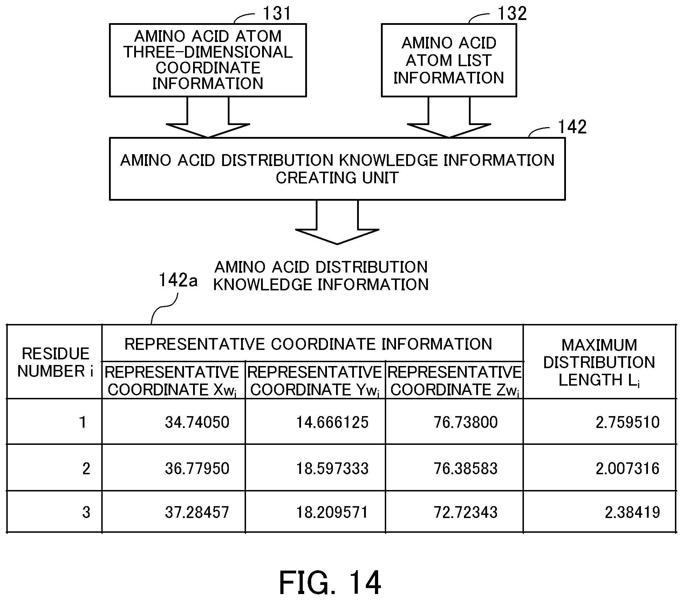

FIG. 14 illustrates an example of amino acid distribution knowledge information. The amino acid distribution knowledge information 142a is created by the amino acid distribution knowledge information creating unit 142 based on the amino acid atom three-dimensional coordinate information 131 and the amino acid atom list information 132.

In the amino acid distribution knowledge information 142a, representative coordinate information and a maximum distribution length "Lᵢ" are set in association with a residue number "i" identifying an amino acid. The representative coordinate of the amino acid having the residue number "i" is represented by coordinate values "Xwᵢ, Ywᵢ, and Zwᵢ" of respective axes in three-dimensional space.

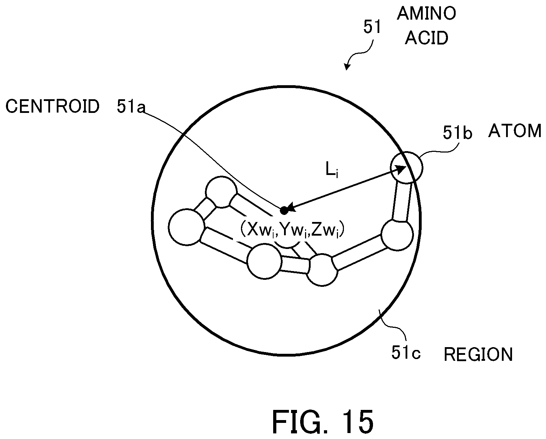

FIG. 15 illustrates an example of a maximum distribution length in a case where the centroid of an amino acid is used as a representative coordinate. When coordinates of a centroid 51a of an amino acid 51 are used as a representative coordinate, a distance between the centroid 51a and an atom 51b located farthest from the centroid 51a among atoms constituting the amino acid 51 becomes the maximum distribution length "Lᵢ". Accordingly, all atoms constituting the amino acid 51 are included within a spherical region 51c having a radius "Lᵢ" centered at the centroid 51a. Further, the radius of the region 51c is a minimum value capable of including all atoms.

FIG. 16 is a flowchart illustrating an example of a procedure of amino acid distribution knowledge information creation processing. The processing illustrated in FIG. 16 will be described below according to step numbers.

[Step S301] The amino acid distribution knowledge information creating unit 142 acquires, from the protein information storing unit 130, list information indicating atoms constituting an amino acid to be processed. When distribution locality is calculated, amino acids to be processed are n amino acids extracted as samples. When exclusion targets are determined, amino acids to be processed are all amino acids included in a protein.

[Step S302] The amino acid distribution knowledge information creating unit 142 calculates representative coordinates of amino acids. The representative coordinates are, for example, coordinates of centroids of the amino acids. Details of representative coordinate calculation processing will be described later (see FIG. 17).

[Step S303] The amino acid distribution knowledge information creating unit 142 calculates maximum distribution lengths of the amino acids. Details of the amino acid maximum distribution length calculation processing will be described later (see FIG. 18).

[Step S304] The amino acid distribution knowledge information creating unit 142 combines, for each amino acid, representative coordinate information and maximum distribution length information, and outputs the combined information as amino acid distribution knowledge information.

FIG. 17 is a flowchart illustrating an example of a procedure of representative coordinate calculation processing for amino acids. The processing illustrated in FIG. 17 will be described below according to step numbers.

[Step S401] The amino acid distribution knowledge information creating unit 142 designates one unprocessed amino acid as a calculation target amino acid.

[Step S402] The amino acid distribution knowledge information creating unit 142 acquires list information of atoms constituting the calculation target amino acid.

[Step S403] The amino acid distribution knowledge information creating unit 142 acquires three-dimensional coordinate information (coordinate values on respective axes in three-dimensional space) of each atom constituting the calculation target amino acid from the amino acid atom three-dimensional coordinate information 131.

[Step S404] The amino acid distribution knowledge information creating unit 142 calculates a centroid of the calculation target amino acid based on the acquired three-dimensional coordinate information. For example, the amino acid distribution knowledge information creating unit 142 acquires a mass number of each atom based on an atom type of each atom, and calculates the centroid of the calculation target amino acid based on mass numbers of respective atoms and the three-dimensional coordinate information. The amino acid distribution knowledge information creating unit 142 may also calculate the centroid in a simplified manner by regarding masses of respective atoms as identical.

[Step S405] The amino acid distribution knowledge information creating unit 142 records the centroid of the calculation target amino acid as a representative coordinate.

[Step S406] The amino acid distribution knowledge information creating unit 142 determines whether representative coordinate calculation processing has been performed for all amino acids to be processed. When an unprocessed amino acid exists, the amino acid distribution knowledge information creating unit 142 advances the processing to step S401. When representative coordinate calculation has been completed for all amino acids to be processed, the amino acid distribution knowledge information creating unit 142 ends the representative coordinate calculation processing for amino acids.

FIG. 18 is a flowchart illustrating an example of a procedure of maximum distribution length calculation processing for amino acids. The processing illustrated in FIG. 18 will be described below according to step numbers.

[Step S501] The amino acid distribution knowledge information creating unit 142 designates one unprocessed amino acid as a calculation target amino acid.

[Step S502] The amino acid distribution knowledge information creating unit 142 acquires list information of atoms constituting the calculation target amino acid.

[Step S503] The amino acid distribution knowledge information creating unit 142 acquires three-dimensional coordinate information of atoms constituting the calculation target amino acid.

[Step S504] The amino acid distribution knowledge information creating unit 142 acquires the representative coordinate of the calculation target amino acid.

[Step S505] The amino acid distribution knowledge information creating unit 142 sets an initial value of the maximum distribution length of the calculation target amino acid to "0".

[Step S506] The amino acid distribution knowledge information creating unit 142 designates one unprocessed atom among atoms constituting the calculation target amino acid as a calculation target atom.

[Step S507] The amino acid distribution knowledge information creating unit 142 calculates a distance between the representative coordinate of the calculation target amino acid and the calculation target atom. The distance is, for example, a Euclidean distance in three-dimensional space.

[Step S508] The amino acid distribution knowledge information creating unit 142 determines whether the distance calculated immediately before is greater than the maximum distribution length. When the distance calculated immediately before is greater than the maximum distribution length, the amino acid distribution knowledge information creating unit 142 advances the processing to step S509. When the distance calculated immediately before is equal to or less than the maximum distribution length, the amino acid distribution knowledge information creating unit 142 advances the processing to step S510.

[Step S509] The amino acid distribution knowledge information creating unit 142 sets the distance calculated immediately before as the maximum distribution length.

[Step S510] The amino acid distribution knowledge information creating unit 142 determines whether distance calculation processing has been completed for all atoms constituting the calculation target amino acid. When processing has been completed for all atoms, the amino acid distribution knowledge information creating unit 142 advances the processing to step S511. When an unprocessed atom exists, the amino acid distribution knowledge information creating unit 142 advances the processing to step S506.

[Step S511] The amino acid distribution knowledge information creating unit 142 determines whether maximum distribution length calculation has been completed for all amino acids to be processed. When processing has been completed for all amino acids, the amino acid distribution knowledge information creating unit 142 ends the maximum distribution length calculation processing for amino acids. When an unprocessed amino acid exists, the amino acid distribution knowledge information creating unit 142 advances the processing to step S501.

When the amino acid distribution knowledge information 142a is obtained, the distribution locality calculating unit 144 performs exclusion determination for each edge definition pair candidate formed by combining two amino acids among the n amino acids. Edge definition pair candidates to be calculated are generated, for example, by the edge definition pair candidate creating unit 141.

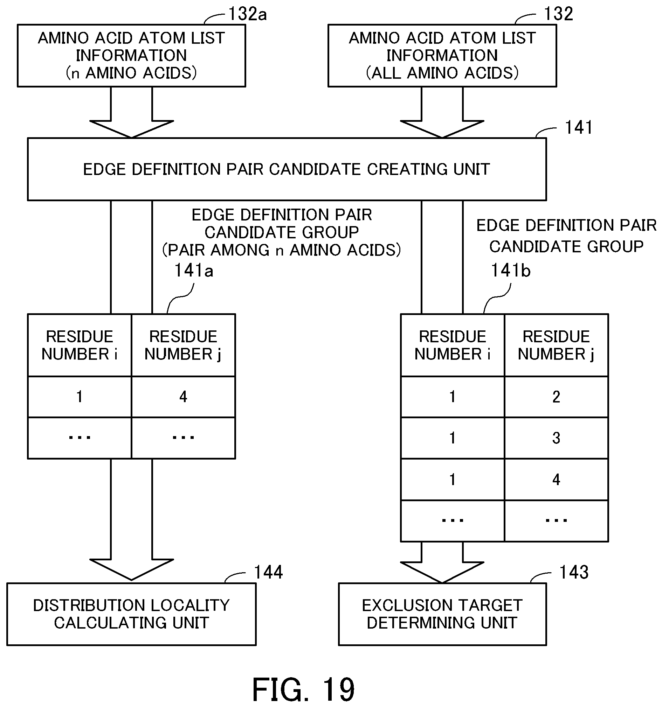

FIG. 19 illustrates an example of edge definition pair candidates. For example, the edge definition pair candidate creating unit 141 creates the edge definition pair candidate group 141a for distribution locality calculation based on amino acid atom list information 132a indicating n amino acids selected from the amino acid atom list information 132. The edge definition pair candidate group 141a for distribution locality calculation is transmitted to the distribution locality calculating unit 144.

The edge definition pair candidate creating unit 141 also creates an edge definition pair candidate group 141b for exclusion target determination based on the amino acid atom list information 132 for all amino acids. The edge definition pair candidate group 141b for exclusion target determination is transmitted to the exclusion target determining unit 143.

In the edge definition pair candidate groups 141a and 141b, pairs of residue numbers corresponding to selected amino acids are registered when two amino acids are selected from amino acids to be processed.

In each of the distribution locality calculating unit 144 and the exclusion target determining unit 143, exclusion determination of edge definition pair candidates is performed based on the input edge definition pair candidate groups 141a and 141b. The exclusion determination is performed by comparing an edge threshold with an inter-residue distance. Hereinafter, exclusion determination processing will be described assuming that the exclusion target determining unit 143 performs the processing; however, in distribution locality calculation processing, the distribution locality calculating unit 144 performs the processing.

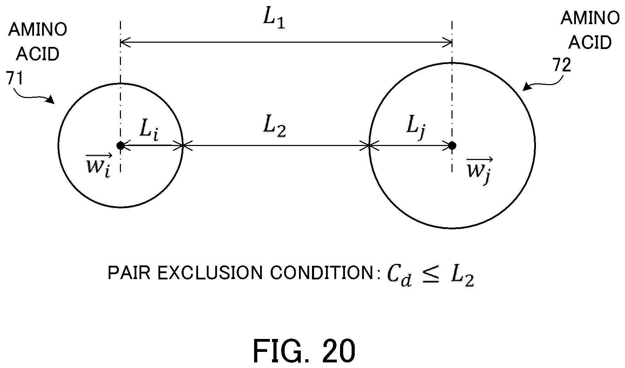

FIG. 20 illustrates a relationship between an edge threshold and an inter-residue distance. For example, assume that there is an edge definition pair candidate between an i-th amino acid 71 and a j-th amino acid 72 (i and j are natural numbers). A representative coordinate of the amino acid 71 is represented by a "vector Wᵢ". A maximum distribution length of the amino acid 71 is "Lᵢ". Similarly, a representative coordinate of the amino acid 72 is represented by a "vector Wⱼ". A maximum distribution length of the amino acid 72 is "Lⱼ".

An inter-representative coordinate distance L₁ between the two amino acids 71 and 72 is expressed as "L₁ - |Wᵢ - Wⱼ|". A lower limit value L₂ of a distance between the two amino acids 71 and 72 is expressed as "L₂ = L₁ - (Lᵢ + Lⱼ)". The lower limit value L₂ indicates that an inter-residue distance between atoms of the amino acids 71 and 72 does not become smaller than the lower limit value L₂ even in a closest case.

A condition for excluding an edge definition pair candidate from a calculation target of inter-residue distance (pair exclusion condition) is, for example, that the lower limit value L₂ of the distance is equal to or greater than a pair threshold C_{d} (C_{d} ≤ L₂). The pair exclusion condition is represented as an exclusion determination expression for edge definition pair candidates using distribution locality information of amino acids. The exclusion determination expression is as follows: $\left|{W}_{i}-{W}_{j}\right| - \left({L}_{i}+{L}_{j}\right) \geq {C}_{d} .$

The edge definition pair candidate exclusion determination expression indicates that, when even the closest possible positions at which atoms of respective amino acids in an amino acid pair may exist exceed the edge threshold, the amino acid pair is excluded from exhaustive distance calculation between all atoms.

The exclusion target determining unit 143 performs determination based on the exclusion determination expression for all edge definition pairs included in the acquired edge definition pair candidate group.

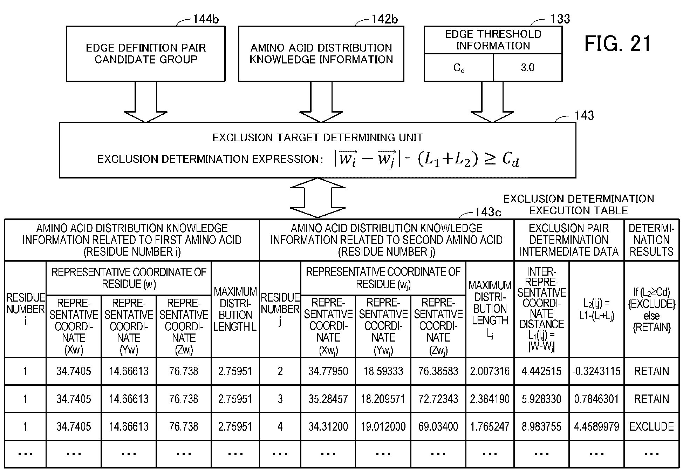

FIG. 21 illustrates an example of exclusion determination processing. The exclusion target determining unit 143 generates an exclusion determination execution table 143c having records corresponding to edge definition pair candidates. For example, the exclusion target determining unit 143 sets amino acid distribution knowledge information corresponding to each edge definition pair candidate included in the edge definition pair candidate group 144b in the same record of the exclusion determination execution table 143c. The exclusion target determining unit 143 calculates the inter-representative coordinate distance L₁ of each edge definition pair candidate and further calculates the lower limit value L₂ of the distance. The exclusion target determining unit 143 registers, in the exclusion determination execution table 143c as exclusion pair determination intermediate data, the calculated inter-representative coordinate distance L₁ and the lower limit value L₂ for each edge definition pair candidate.

Then, the exclusion target determining unit 143 compares the lower limit value L₂ of the distance with the edge threshold C_{d}, and when the lower limit value L₂ of the distance is equal to or greater than the edge threshold C_{d}, determines a determination result of the corresponding edge definition pair candidate as "exclude". When the lower limit value L₂ of the distance is less than the edge threshold C_{d}, the exclusion target determining unit 143 determines a determination result of the corresponding edge definition pair candidate as "retain".

The exclusion target determining unit 143 outputs the exclusion target edge definition pair candidate group 143a and the retention target edge definition pair candidate group 143b based on the determination results.

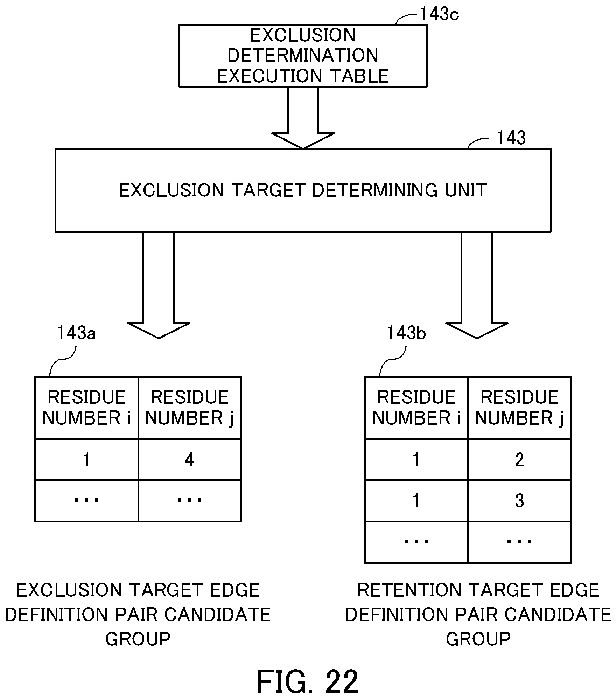

FIG. 22 illustrates an example of an exclusion target edge definition pair candidate group and a retention target edge definition pair candidate group. The exclusion target determining unit 143 defines a set of edge definition pair candidates determined as "exclude" in the determination results indicated in the exclusion determination execution table 143c as the exclusion target edge definition pair candidate group 143a. The exclusion target determining unit 143 also defines a set of edge definition pair candidates determined as "retain" in the determination results indicated in the exclusion determination execution table 143c as the retention target edge definition pair candidate group 143b.

Next, a procedure of exclusion determination processing for edge definition pair candidates will be described in detail.

FIG. 23 is a flowchart illustrating an example of a procedure of exclusion determination processing for edge definition pair candidates. The processing illustrated in FIG. 23 will be described below according to step numbers.

[Step S601] The edge definition pair candidate creating unit 141 acquires list information of atoms constituting each amino acid to be processed.

[Step S602] The edge definition pair candidate creating unit 141 creates edge definition pair candidates by combining residue numbers of amino acids. The edge definition pair candidate creating unit 141 transmits the edge definition pair candidate group 144b including a plurality of edge definition pair candidates to the exclusion target determining unit 143.

[Step S603] The exclusion target determining unit 143 designates one unprocessed edge definition pair candidate as a calculation target edge definition pair candidate.

[Step S604] The exclusion target determining unit 143 acquires representative coordinates " (Xwᵢ, Ywᵢ, Zwᵢ) , (Xwⱼ, Ywⱼ, Zwⱼ)" and maximum distribution lengths "Lᵢ, Lⱼ" of respective pairs of amino acids indicated in the calculation target edge definition pair candidate.

[Step S605] The exclusion target determining unit 143 calculates the inter-representative coordinate distance L₁. The distance L₁ is, for example, "L₁ = {(Xwᵢ - Xwⱼ)² + (Ywᵢ - Ywⱼ)² + (Zwᵢ - Zwⱼ)²}1/2".

[Step S606] The exclusion target determining unit 143 calculates the lower limit value L₂ of the distance. The lower limit value L₂ of the distance is, for example, "L₂ = L₁ - (Lᵢ + Lⱼ)".

[Step S607] The exclusion target determining unit 143 determines whether the lower limit value L₂ of the distance is equal to or greater than the edge threshold C_{d}. When the lower limit value L₂ of the distance is equal to or greater than the edge threshold C_{d}, the exclusion target determining unit 143 advances the processing to step S609. When the lower limit value L₂ of the distance is less than the edge threshold C_{d}, the exclusion target determining unit 143 advances the processing to step S608.

[Step S608] The exclusion target determining unit 143 records a determination result of the calculation target edge definition pair candidate as "retain". The exclusion target determining unit 143 then advances the processing to step S610.

[Step S609] The exclusion target determining unit 143 records a determination result of the calculation target edge definition pair candidate as "exclude".

[Step S610] The exclusion target determining unit 143 determines whether all edge definition pair candidates to be processed have been processed. When processing of all edge definition pair candidates has been completed, the exclusion target determining unit 143 advances the processing to step S611. When an unprocessed edge definition pair candidate exists, the exclusion target determining unit 143 advances the process to step S603.

[Step S611] The exclusion target determining unit 143 outputs the exclusion target edge definition pair candidate group 143a including edge definition pair candidates determined as "exclude" and the retention target edge definition pair candidate group 143b including edge definition pair candidates determined as "retain".

Thus, the edge definition pair candidate group 144b is divided by the exclusion target determining unit 143 into the exclusion target edge definition pair candidate group 143a and the retention target edge definition pair candidate group 143b. The exclusion target determining unit 143 performs exclusion determination processing when the exclusion appropriateness determining unit 145 has determined that edge definition pair candidates included in the exclusion target edge definition pair candidate group are to be excluded from inter-residue distance calculation targets.

When the exclusion target determining unit 143 performs exclusion determination processing, the inter-node edge connecting unit 146 calculates inter-residue distances for edge definition pair candidates indicated in the retention target edge definition pair candidate group 143b and determines whether edges between nodes corresponding to amino acids are connected. That is, the inter-node edge connecting unit 146 determines that node connection is not performed for amino acids whose lower limit value L₂ of the distance is equal to or greater than the edge threshold C_{d} without performing inter-residue distance calculation. The inter-node edge connecting unit 146 calculates inter-residue distances for amino acids whose lower limit value L₂ of the distance is less than the edge threshold C_{d} and determines that edge connection is performed when the inter-residue distance is equal to or less than the edge threshold C_{d}.

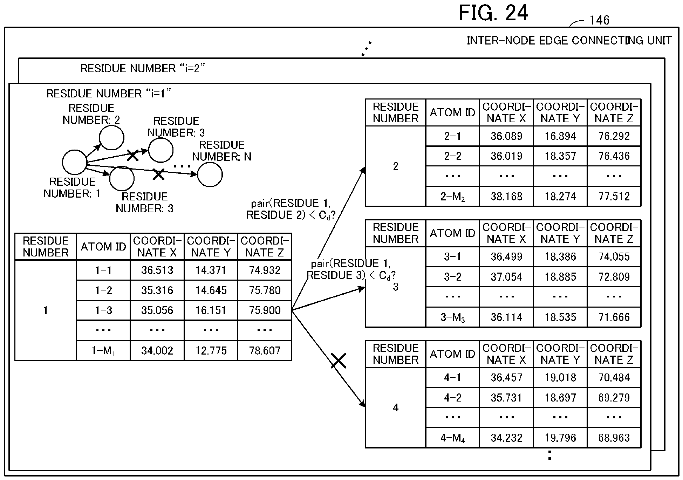

FIG. 24 illustrates an example of processing for determining whether an edge connection is established based on an inter-residue distance. For example, as illustrated in FIG. 22, it is assumed that an edge definition pair candidate between an amino acid having residue number "1" and an amino acid having residue number "2" is a retention target, and that an edge definition pair candidate between an amino acid having residue number "1" and an amino acid having residue number "3" is also a retention target. It is also assumed that an edge definition pair candidate between an amino acid having residue number "1" and an amino acid having residue number "4" is an exclusion target.

In this case, the inter-node edge connecting unit 146 calculates distances between all atoms for the amino acid having residue number "1" and the amino acid having residue number "2". Specifically, the inter-node edge connecting unit 146 generates all possible atom pairs by selecting one atom from atoms constituting the amino acid having residue number "1" and selecting one atom from atoms constituting the amino acid having residue number "2". The inter-node edge connecting unit 146 calculates distances for all generated atom pairs. The inter-node edge connecting unit 146 sets a minimum value among distances for respective atom pairs as a distance between amino acids (inter-residue distance).

Similarly, the inter-node edge connecting unit 146 calculates distances between all atoms for the amino acid having residue number "1" and the amino acid having residue number "3". The inter-node edge connecting unit 146 sets a minimum value among distances between atoms as the inter-residue distance.

The inter-node edge connecting unit 146 does not calculate distances between atoms for the amino acid having residue number "1" and the amino acid having residue number "4". That is, the inter-node edge connecting unit 146 does not generate an edge connecting the amino acid having residue number "1" and the amino acid having residue number "4".

When the inter-node edge connecting unit 146 calculates an inter-residue distance of an edge definition pair candidate, if the inter-residue distance is smaller than the edge threshold C_{d}, the inter-node edge connecting unit 146 determines that the edge definition pair candidate becomes an edge definition pair to be connected.

FIG. 24 illustrates a calculation state of inter-residue distances between the amino acid having residue number "1" and other amino acids. However, inter-residue distances between amino acids having residue numbers "2, 3, ..." and other amino acids are also calculated for respective residue numbers. Nevertheless, calculation of inter-residue distances is performed only when an edge definition pair candidate is a retention target.

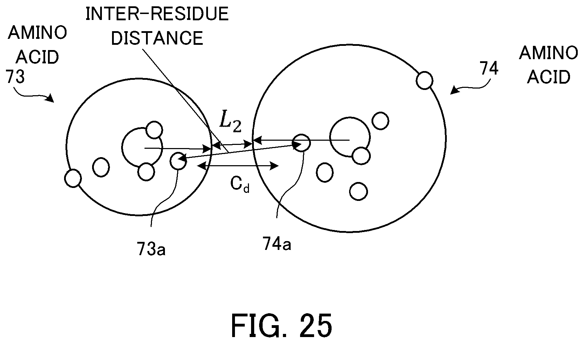

FIG. 25 illustrates an example of an inter-residue distance between amino acids. The lower limit value L₂ of a distance between two amino acids 73 and 74 is smaller than the edge threshold C_{d}. Therefore, an edge definition pair candidate corresponding to a pair of the amino acids 73 and 74 becomes a calculation target for an inter-residue distance.

Accordingly, the inter-node edge connecting unit 146 calculates distances between atoms by exhaustive calculation of distances between atoms in the amino acid 73 and atoms in the amino acid 74, and sets a minimum value of the distances as the inter-residue distance. As a result, the inter-residue distance is calculated strictly.

In the example of FIG. 25, a distance between an atom 73a of the amino acid 73 and an atom 74a of the amino acid 74 is a shortest distance between atoms in the two amino acids 73 and 74. That is, the distance becomes the inter-residue distance. The inter-residue distance is larger than the edge threshold C_{d}. Therefore, an edge definition pair candidate corresponding to the amino acids 73 and 74 is excluded from edge definition pairs. As described above, even when the lower limit value L₂ of the distance is smaller than the edge threshold C_{d}, the inter-residue distance may become equal to or larger than the edge threshold C_{d}.

The inter-node edge connecting unit 146 calculates inter-residue distances for all edge definition pair candidates included in the retention target edge definition pair candidate group 143b and determines whether edges are defined. The inter-node edge connecting unit 146 outputs information of defined edges as graph information.

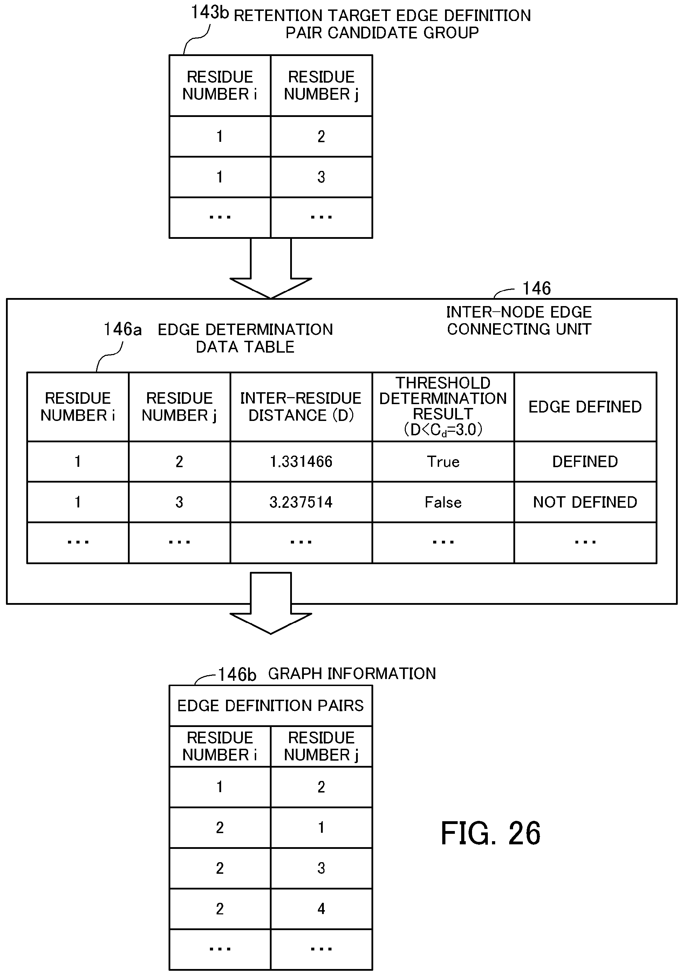

FIG. 26 illustrates an example of graph information output by edge generation processing. For example, the inter-node edge connecting unit 146 manages a result of the edge generation processing by using an edge determination data table 146a. In the edge determination data table 146a, an inter-residue distance, a threshold determination result, and whether an edge is defined are set in association with a pair of residue numbers serving as an edge definition pair candidate.

The inter-node edge connecting unit 146 extracts pairs of residue numbers of edge definition pair candidates determined as "defined" in the edge defined result, and registers the extracted pairs as edge definition pairs in graph information 146b. A graph representing relationships between amino acids in which interactions occur is generated by connecting nodes corresponding to amino acids indicated by the edge definition pairs registered in the graph information 146b with edges.

FIG. 27 is a flowchart illustrating an example of a procedure of edge generation processing. The processing illustrated in FIG. 27 will be described below according to step numbers.

[Step S701] The inter-node edge connecting unit 146 acquires the retention target edge definition pair candidate group 143b.

[Step S702] The inter-node edge connecting unit 146 designates, from the retention target edge definition pair candidate group 143b, one unprocessed edge definition pair candidate as a calculation target.

[Step S703] The inter-node edge connecting unit 146 calculates a strict inter-residue distance D by exhaustive calculation of distances between atoms.

[Step S704] The inter-node edge connecting unit 146 determines whether the inter-residue distance D is smaller than the edge threshold C_{d}. When the inter-residue distance D is smaller, the inter-node edge connecting unit 146 advances the processing to step S705. When the inter-residue distance D is equal to or greater than the edge threshold C_{d}, the inter-node edge connecting unit 146 advances the processing to step S706.

[Step S705] The inter-node edge connecting unit 146 sets the calculation target edge definition pair candidate as an edge definition pair. Thereafter, the inter-node edge connecting unit 146 advances the processing to step S707.

[Step S706] The inter-node edge connecting unit 146 excludes the calculation target edge definition pair candidate from edge definition pairs.

[Step S707] The inter-node edge connecting unit 146 determines whether all edge definition pair candidates included in the retention target edge definition pair candidate group 143b have been processed as calculation targets. When processing for all edge definition pair candidates has been completed, the inter-node edge connecting unit 146 advances the processing to step S708. When an unprocessed edge definition pair candidate remains, the inter-node edge connecting unit 146 advances the processing to step S702.

[Step S708] The inter-node edge connecting unit 146 outputs the graph information 146b. That is, the inter-node edge connecting unit 146 extracts edge definition pair candidates set as edge definition pairs from the retention target edge definition pair candidate group 143b and outputs the graph information 146b listing the extracted edge definition pairs. The output graph information 146b represents a graph of a protein.

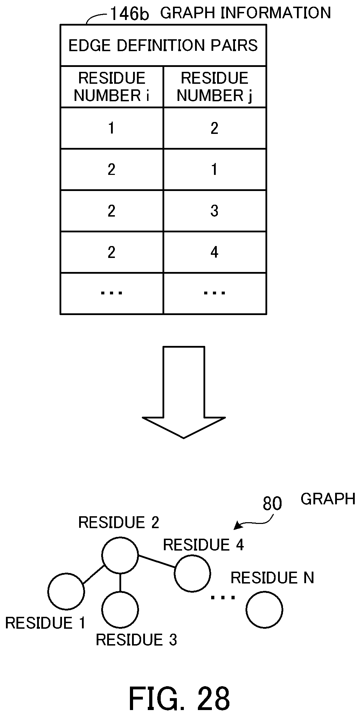

FIG. 28 illustrates an example of a graph. In a graph 80, nodes corresponding to respective amino acids constituting a protein are provided. Each node is identified by a residue number assigned to the corresponding amino acid in the protein. In edge definition pairs set in the graph information 146b, pairs of residue numbers are indicated. In the graph 80, nodes corresponding to respective residue numbers indicated in edge definition pairs are connected by edges.

In this manner, the graph 80 is obtained. When the lower limit value L₂ of a distance between amino acids is equal to or greater than the edge threshold C_{d} during generation of the graph 80, calculation of a shortest distance by exhaustive calculation between atoms of the amino acids is not performed. As a result, efficient graph generation is achieved.

When locality of distribution of atoms constituting amino acids is small, a ratio of amino-acid pairs in which the lower limit value L₂ of a distance between amino acids is equal to or greater than the edge threshold C_{d} becomes small. Therefore, when sampling reveals that the ratio of amino-acid pairs in which the lower limit value L₂ of the distance between the amino acids is equal to or greater than the edge threshold C_{d} is small, shortest-distance calculation by exhaustive calculation is performed for all amino-acid pairs. Accordingly, when an effect obtained by suppressing calculation of shortest distances between amino acids whose lower limit value L₂ of the distance between the amino acids is equal to or greater than the edge threshold C_{d} is not expected, processing for calculating the lower limit value L₂ for all amino-acid pairs is suppressed. As a result, an increase in overall processing load is prevented, such increase being caused when a calculation amount for the lower limit value L₂ for all amino-acid pairs exceeds a reduction effect of calculation cost.

### [Other Embodiments]

In the second embodiment, coordinates of a centroid are used as a representative coordinate; however, coordinates other than a centroid may also be used as a representative coordinate. For example, the graph generating unit 140 may use coordinates of CA, which is always included in each amino acid, as a representative coordinate. In addition, the graph generating unit 140 may use, instead of a centroid obtained by an arithmetic mean of coordinates of atoms constituting each amino acid, a geometric mean (product mean) or a harmonic mean. Further, the graph generating unit 140 may use coordinates of a median value as a representative coordinate instead of a centroid obtained by an arithmetic mean of coordinates of atoms constituting each amino acid.

Furthermore, the graph generating unit 140 may select one atom among atoms constituting each amino acid and may use coordinates of the selected atom as a representative coordinate. For example, the graph generating unit 140 may use coordinates of one atom selected at random from atoms constituting an amino acid as a representative coordinate. The graph generating unit 140 may also use coordinates of an atom in a predetermined order based on an order of atom IDs (for example, an atom having the smallest ID) as a representative coordinate.

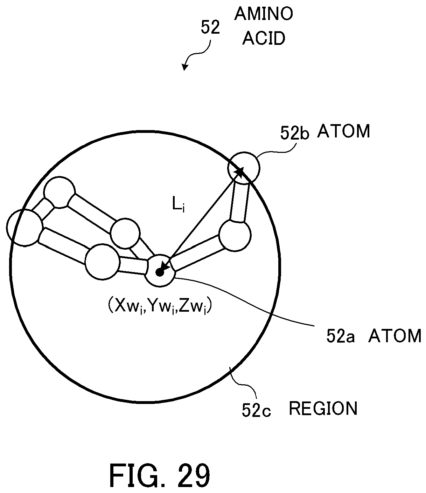

FIG. 29 illustrates an example of a maximum distribution length when coordinates of a predetermined atom are used as a representative coordinate. When coordinates of a predetermined atom 52a in an amino acid 52 are used as a representative coordinate, a distance between the atom 52a and an atom 52b farthest from the atom 52a among atoms constituting the amino acid 52 becomes the maximum distribution length "Lᵢ". Accordingly, all atoms constituting the amino acid 52 are included within a spherical region 52c having a radius "Lᵢ" centered on the atom 52a. The radius of the region 52c is a minimum value that allows inclusion of all atoms.

The above description merely illustrates principles of the present disclosure. In addition, many modifications and variations are possible for those skilled in the art, and the present disclosure is not limited to the exact configurations and application examples illustrated and described above. All corresponding modifications and equivalents are regarded as falling within the scope defined by the appended claims and equivalents thereof.

### Reference Signs List

1 protein
1a to 1c amino acid
2a to 2c region
3 graph
3a to 3c node
3d edge
10 information processing apparatus
11 storing unit
11a protein information
12 processing unit

## Claims

1. An information processing program for determining whether a predetermined relationship exists between a plurality of amino acids included in a protein, each of the plurality of amino acids being composed of a plurality of atoms, the information processing program causing a computer to execute a process comprising:
calculating, for each of a plurality of amino acid pairs each being a combination of two amino acids among the plurality of amino acids, a distance between the combined amino acids;
determining, in response to the distance being equal to or greater than a threshold, that the predetermined relationship does not exist between the combined amino acids; and
calculating, in response to the distance being less than the threshold, an inter-atomic distance between an atom constituting one amino acid of the combined amino acids and an atom constituting another amino acid of the combined amino acids, and determining, based on the inter-atomic distance, whether the predetermined relationship exists between the combined amino acids.

2. The information processing program according to claim 1, wherein the process further includes:
determining, in a graph having nodes each corresponding to a respective one of the plurality of amino acids and edges each connecting nodes between which the predetermined relationship exists, not to connect, by the edge, nodes corresponding to the combined amino acids constituting a first amino acid pair for which the distance between the combined amino acids is equal to or greater than the threshold.

3. The information processing program according to claim 2, wherein the process further includes:
calculating, for each of the plurality of amino acids, based on protein information regarding the protein, a first region including atoms constituting the amino acid, the protein information including information regarding the plurality of amino acids and information regarding positions of the plurality of atoms constituting the plurality of amino acids;
calculating, for each of the plurality of amino acid pairs, a gap distance between the first regions of the combined amino acids; and
determining, in the graph, not to connect, by the edge, the nodes corresponding to the combined amino acids constituting the first amino acid pair for which the gap distance between the first regions is equal to or greater than the threshold.

4. The information processing program according to claim 2, wherein the process further includes:
calculating representative coordinates of the plurality of amino acids based on positions of the plurality of atoms constituting the plurality of amino acids;
calculating, for each of the plurality of amino acid pairs, a distance between the representative coordinates of the combined amino acids; and
determining, in the graph, not to connect, by the edge, the nodes corresponding to the combined amino acids constituting the first amino acid pair for which the distance between the representative coordinates of the combined amino acids is equal to or greater than the threshold.

5. The information processing program according to claim 3, wherein the calculating of the first region includes calculating a representative coordinate of a calculation-target amino acid based on positions of the plurality of atoms constituting the calculation-target amino acid, and calculating the first region centered at the representative coordinate and including the plurality of atoms constituting the calculation-target amino acid, the first region being spherical.

6. The information processing program according to claim 5, wherein the calculating of the first region includes determining a radius of the first region based on a maximum distance from the representative coordinate to the plurality of atoms constituting the calculation-target amino acid.

7. The information processing program according to claim 6, wherein the process further includes:
calculating, for a second amino acid pair for which the gap distance between the first regions is less than the threshold, inter-atomic distances between each of the plurality of atoms constituting a first amino acid of the second amino acid pair and each of the plurality of atoms constituting a second amino acid of the second amino acid pair based on positions of the plurality of atoms constituting the first amino acid and positions of the plurality of atoms constituting the second amino acid;
calculating a shortest distance between the first amino acid and the second amino acid based on calculation results of the inter-atomic distances between the first amino acid and the second amino acid;
determining, in response to the shortest distance between the first amino acid and the second amino acid being equal to or greater than the threshold, not to connect, by the edge, a first node corresponding to the first amino acid and a second node corresponding to the second amino acid in the graph; and
determining, in response to the shortest distance between the first amino acid and the second amino acid being less than the threshold, to connect, by the edge, the first node and the second node in the graph.

8. The information processing program according to claim 3, wherein the process further includes:
extracting some of the plurality of amino acid pairs as sampling-target amino acid pairs;
calculating, for each sampling-target amino acid included in one of the sampling-target amino acid pairs, a second region including the plurality of atoms constituting the sampling-target amino acid;
calculating, for each of the sampling-target amino acid pairs, a gap distance between the second regions of the respective combined amino acids;
calculating, among the sampling-target amino acid pairs, a ratio of sampling-target amino acid pairs for which the gap distance between the second regions is less than the threshold;
suppressing, in response to the ratio exceeding a predetermined value, the calculating of the first region, the calculating of the gap distance between the first regions, and the determining of not to connect, by the edge, the nodes corresponding to the combined amino acids constituting the first amino acid pair for which the gap distance between the first regions is equal to or greater than the threshold;
calculating, for each of the plurality of amino acid pairs as calculation targets, in response to the ratio exceeding the predetermined value, inter-atomic distances between each of the plurality of atoms constituting a third amino acid of the calculation-target amino acid pair and each of the plurality of atoms constituting a fourth amino acid of the calculation-target amino acid pair based on positions of the plurality of atoms constituting the third amino acid and positions of the plurality of atoms constituting the fourth amino acid;
calculating a shortest distance between the third amino acid and the fourth amino acid based on calculation results of the inter-atomic distances between the third amino acid and the fourth amino acid;
determining, in response to the shortest distance between the third amino acid and the fourth amino acid being equal to or greater than the threshold, not to connect, by the edge, a third node corresponding to the third amino acid and a fourth node corresponding to the fourth amino acid in the graph; and
determining, in response to the shortest distance between the third amino acid and the fourth amino acid being less than the threshold, to connect, by the edge, the third node and the fourth node in the graph.

9. An information processing method executed by a computer for determining whether a predetermined relationship exists between a plurality of amino acids included in a protein, each of the plurality of amino acids being composed of a plurality of atoms, the information processing method comprising:
calculating, for each of a plurality of amino acid pairs each being a combination of two amino acids among the plurality of amino acids, a distance between the combined amino acids;
determining, in response to the distance being equal to or greater than a threshold, that the predetermined relationship does not exist between the combined amino acids; and
calculating, in response to the distance being less than the threshold, an inter-atomic distance between an atom constituting one amino acid of the combined amino acids and an atom constituting another amino acid of the combined amino acids, and determining, based on the inter-atomic distance, whether the predetermined relationship exists between the combined amino acids.

10. An information processing apparatus for determining whether a predetermined relationship exists between a plurality of amino acids included in a protein, each of the plurality of amino acids being composed of a plurality of atoms, the information processing apparatus comprising:
processing means for:
calculating, for each of a plurality of amino acid pairs each being a combination of two amino acids among the plurality of amino acids, a distance between the combined amino acids,
determining, in response to the distance being equal to or greater than a threshold, that the predetermined relationship does not exist between the combined amino acids, and
calculating, in response to the distance being less than the threshold, an inter-atomic distance between an atom constituting one amino acid of the combined amino acids and an atom constituting another amino acid of the combined amino acids, and determining, based on the inter-atomic distance, whether the predetermined relationship exists between the combined amino acids.
